(19) Europäisches Patentamt
European Patent Office
Office européen des brevets

(11) EP 4 708 147 A2

(12) EUROPEAN PATENT APPLICATION

(43) Date of publication:
11.03.2026 Bulletin 2026/11

(51) International Patent Classification (IPC):
G06N 3/084 (2023.01)

(21) Application number: 25216058.5

(22) Date of filing: 23.11.2021

(52) Cooperative Patent Classification (CPC):
G16B 15/30; G06N 3/045; G06N 3/0499;
G06N 3/084; G06N 3/09; G16B 15/20; G16B 40/20

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 28.11.2020 US 202063118917 P
11.05.2021 US 202163187362 P

(62) Document number(s) of the earlier application(s) in
accordance with Art. 76 EPC:
21816053.9 / 4 200 854

(71) Applicant: GDM Holding LLC
Mountain View, CA 94043 (US)

(72) Inventors:
• FIGURNOV, Mikhail
London N1C 4AG (GB)
• PRITZEL, Alexander
London N1C 4AG (GB)

(74) Representative: Marks & Clerk GST
1 New York Street
Manchester M1 4HD (GB)

Remarks:
This application was filed on 14-11-2025 as a
divisional application to the application mentioned
under INID code 62.

(54) **PREDICTING PROTEIN STRUCTURES BY SHARING INFORMATION BETWEEN MULTIPLE SEQUENCE ALIGNMENTS AND PAIR EMBEDDINGS**

(57) Methods, systems, and apparatus, including computer programs encoded on a computer storage medium, for predicting protein structure. An initial single embedding and initial structure parameters are obtained for each amino acid. A folding neural network, comprising multiple update blocks, processes these inputs to generate final structure parameters that characterize the predicted structure. Each update block is configured to receive an input comprising final pair embeddings, a current single embedding, and current structure parameters for each amino acid. The block processes this input to update the current single embedding and structure parameters. This iterative refinement yields the final structure parameters. The resulting predicted protein structure is then used to evaluate interactions with one or more candidate ligands, and one or more ligands are selected based on the evaluation.

FOLDING NEURAL NETWORK 600

FIG. 6

EP 4 708 147 A2

**Description**

CROSS-REFERENCE TO RELATED APPLICATIONS

This application claims the benefit of the filing date of U.S. Provisional Patent Application Serial No. 63/118,917, which was filed on November 28, 2020, and U.S. Provisional Patent Application Serial No. 63/187,362, which was filed on May 11, 2021, and which are incorporated herein by reference in their entirety.

BACKGROUND

This specification relates to predicting protein structures.

A protein is specified by one or more sequences ("chains") of amino acids. An amino acid is an organic compound which includes an amino functional group and a carboxyl functional group, as well as a side chain (i.e., group of atoms) that is specific to the amino acid. Protein folding refers to a physical process by which one or more sequences of amino acids fold into a three-dimensional (3-D) configuration. The structure of a protein defines the 3-D configuration of the atoms in the amino acid sequences of the protein after the protein undergoes protein folding. When in a sequence linked by peptide bonds, the amino acids may be referred to as amino acid residues.

Predictions can be made using machine learning models. Machine learning models receive an input and generate an output, e.g., a predicted output, based on the received input. Some machine learning models are parametric models and generate the output based on the received input and on values of the parameters of the model. Some machine learning models are deep models that employ multiple layers of models to generate an output for a received input. For example, a deep neural network is a deep machine learning model that includes an output layer and one or more hidden layers that each apply a non-linear transformation to a received input to generate an output.

SUMMARY

This specification describes a neural network system implemented as computer programs on one or more computers in one or more locations for predicting protein structures.

As used throughout this specification, the term "protein" can be understood to refer to any biological molecule that is specified by one or more sequences (or "chains") of amino acids. For example, the term protein can refer to a protein domain, e.g., a portion of an amino acid chain of a protein that can undergo protein folding nearly independently of the rest of the protein. As another example, the term protein can refer to a protein complex, i.e., that includes multiple amino acid chains that jointly fold into a protein structure.

A "multiple sequence alignment" (MSA) for an amino acid chain in a protein specifies a sequence alignment of the amino acid chain with multiple additional amino acid chains, e.g., from other proteins, e.g., homologous proteins. More specifically, the MSA can define a correspondence between the positions in the amino acid chain and corresponding positions in multiple additional amino acid chains. A MSA for an amino acid chain can be generated, e.g., by processing a database of amino acid chains using any appropriate computational sequence alignment technique, e.g., progressive alignment construction. The amino acid chains in the MSA can be understood as having an evolutionary relationship, e.g., where each amino acid chain in the MSA may share a common ancestor. The correlations between the amino acids in the amino acid chains in a MSA for an amino acid chain can encode information that is relevant to predicting the structure of the amino acid chain.

An "embedding" of an entity (e.g., a pair of amino acids) can refer to a representation of the entity as an ordered collection of numerical values, e.g., a vector or matrix of numerical values.

The structure of a protein can be defined by a set of structure parameters. A set of structure parameters defining the structure of a protein can be represented as an ordered collection of numerical values. A few examples of possible structure parameters for defining the structure of a protein are described in more detail next.

In one example, the structure parameters defining the structure of a protein include: (i) location parameters, and (ii) rotation parameters, for each amino acid in the protein.

The location parameters for an amino acid can specify a predicted 3-D spatial location of a specified atom in the amino acid in the structure of the protein. The specified atom can be the alpha carbon atom in the amino acid, i.e., the carbon atom in the amino acid to which the amino functional group, the carboxyl functional group, and the side chain are bonded. The location parameters for an amino acid can be represented in any appropriate coordinate system, e.g., a three-dimensional $[x, y, z]$ Cartesian coordinate system.

The rotation parameters for an amino acid can specify the predicted "orientation" of the amino acid in the structure of the protein. More specifically, the rotation parameters can specify a 3-D spatial rotation operation that, if applied to the coordinate system of the location parameters, causes the three "main chain" atoms in the amino acid to assume fixed positions relative to the rotated coordinate system. The three main chain atoms in the amino acid can refer to the linked series of nitrogen, alpha carbon, and carbonyl carbon atoms in the amino acid. The rotation parameters for an amino acid can be represented, e.g., as an orthonormal $3 \times 3$ matrix with determinant equal to 1.

Generally, the location and rotation parameters for an amino acid define an egocentric reference frame for the amino acid. In this reference frame, the side chain for each amino acid may start at the origin, and the first bond along the side chain (i.e., the alpha carbon - beta carbon bond) may be along a defined direction.

In another example, the structure parameters defining the structure of a protein can include a "distance map" that characterizes a respective estimated distance (e.g., measured in angstroms) between each pair of amino acids in the protein. A distance map can characterize the estimated distance between a pair of amino acids, e.g., by a probability distribution over a set of possible distances between the pair of amino acids.

In another example, the structure parameters defining the structure of a protein can define a three-dimensional (3D) spatial location of each atom in each amino acid in the structure of the protein.

The protein structure prediction system described herein can be used to obtain a ligand such as a drug or a ligand of an industrial enzyme. For example, a method of obtaining a ligand may include obtaining a target amino acid sequence, in particular the amino acid sequence of a target protein, e.g. a drug target, and processing an input based on the target amino acid sequence using the protein structure prediction system to determine a (tertiary) structure of the target protein, i.e., the predicted protein structure. The method may then include evaluating an interaction of one or more candidate ligands with the structure of the target protein. The method may further include selecting one or more of the candidate ligands as the ligand dependent on a result of the evaluating of the interaction.

In some implementations, evaluating the interaction may include evaluating binding of the candidate ligand with the structure of the target protein. For example, evaluating the interaction may include identifying a ligand that binds with sufficient affinity for a biological effect. In some other implementations, evaluating the interaction may include evaluating an association of the candidate ligand with the structure of the target protein which has an effect on a function of the target protein, e.g., an enzyme. The evaluating may include evaluating an affinity between the candidate ligand and the structure of the target protein, or evaluating a selectivity of the interaction. The candidate ligand(s) may be selected according to which have the highest affinity.

The candidate ligand(s) may be derived from a database of candidate ligands, and/or may be derived by modifying ligands in a database of candidate ligands, e.g., by modifying a structure or amino acid sequence of a candidate ligand, and/or may be derived by stepwise or iterative assembly/optimization of a candidate ligand.

The evaluation of the interaction of a candidate ligand with the structure of the target protein may be performed using a computer-aided approach in which graphical models of the candidate ligand and target protein structure are displayed for user-manipulation, and/or the evaluation may be performed partially or completely automatically, for example using standard molecular (protein-ligand) docking software. In some implementations the evaluation may include determining an interaction score for the candidate ligand, where the interaction score includes a measure of an interaction between the candidate ligand and the target protein. The interaction score may be dependent upon a strength and/or specificity of the interaction, e.g., a score dependent on binding free energy. A candidate ligand may be selected dependent upon its score.

In some implementations the target protein includes a receptor or enzyme and the ligand is an agonist or antagonist of the receptor or enzyme. In some implementations the method may be used to identify the structure of a cell surface marker. This may then be used to identify a ligand, e.g., an antibody or a label such as a fluorescent label, which binds to the cell surface marker. This may be used to identify and/or treat cancerous cells.

In some implementations the ligand is a drug and the predicted structure of each of a plurality of target proteins is determined, and the interaction of the one or more candidate ligands with the predicted structure of each of the target proteins is evaluated. Then one or more of the candidate ligands may be selected either to obtain a ligand that (functionally) interacts with each of the target proteins, or to obtain a ligand that (functionally) interacts with only one of the target proteins. For example in some implementations it may be desirable to obtain a drug that is effective against multiple drug targets. Also or instead it may be desirable to screen a drug for off-target effects. For example in agriculture it can be useful to determine that a drug designed for use with one plant species does not interact with another, different plant species and/or an animal species.

In some implementations the candidate ligand(s) may include small molecule ligands, e.g., organic compounds with a molecular weight of <900 daltons. In some other implementations the candidate ligand(s) may include polypeptide ligands, i.e., defined by an amino acid sequence.

In some cases, the protein structure prediction system can be used to determine the structure of a candidate polypeptide ligand, e.g., a drug or a ligand of an industrial enzyme. The interaction of this with a target protein structure may then be evaluated; the target protein structure may have been determined using a structure prediction neural network or using conventional physical investigation techniques such as x-ray crystallography and/or magnetic resonance techniques or cryogenic electron microscopy.

In another aspect there is provided a method of using a protein structure prediction system to obtain a polypeptide ligand (e.g., the molecule or its sequence). The method may include obtaining an amino acid sequence of one or more candidate polypeptide ligands. The method may further include using the protein structure prediction system to determine (tertiary) structures of the candidate polypeptide ligands. The method may further include obtaining a target protein structure of a target protein, in silico and/or by physical investigation, and evaluating an interaction between the structure of each of the one or more candidate polypeptide ligands and the target protein structure. The method may further include selecting one or more of the candidate polypeptide ligands as the polypeptide ligand dependent on a result of the evaluation.

As before evaluating the interaction may include evaluating binding of the candidate polypeptide ligand with the structure of the target protein, e.g., identifying a ligand that binds with sufficient affinity for a biological effect, and/or evaluating an association of the candidate polypeptide ligand with the structure of the target protein which has an effect on a function of the target protein, e.g., an enzyme, and/or evaluating an affinity between the candidate polypeptide ligand and the structure of the target protein, or evaluating a selectivity of the interaction. In some implementations the polypeptide ligand may be an aptamer. Again the polypeptide candidate ligand(s) may be selected according to which have the highest affinity.

As before the selected polypeptide ligand may comprise a receptor or enzyme and the ligand may be an agonist or antagonist of the receptor or enzyme. In some implementations the polypeptide ligand may comprises an antibody and the target protein comprises an antibody target, for example a virus, in particular a virus coat protein, or a protein expressed on a cancer cell. In these implementations the antibody binds to the antibody target to provide a therapeutic effect. For example, the antibody may bind to the target and act as an agonist for a particular receptor; alternatively, the antibody may prevent binding of another ligand to the target, and hence prevent activation of a relevant biological pathway.

Implementations of the method may further include synthesizing, i.e., making, the small molecule or polypeptide ligand. The ligand may be synthesized by any conventional chemical techniques and/or may already be available, e.g., may be from a compound library or may have been synthesized using combinatorial chemistry.

The method may further include testing the ligand for biological activity in vitro and/or in vivo. For example the ligand may be tested for ADME (absorption, distribution, metabolism, excretion) and/or toxicological properties, to screen out unsuitable ligands. The testing may include, e.g., bringing the candidate small molecule or polypeptide ligand into contact with the target protein and measuring a change in expression or activity of the protein.

In some implementations a candidate (polypeptide) ligand may include: an isolated antibody, a fragment of an isolated antibody, a single variable domain antibody, a bi- or multi-specific antibody, a multivalent antibody, a dual variable domain antibody, an immunoconjugate, a fibronectin molecule, an adnectin, an DARPin, an avimer, an affibody, an anticalin, an affilin, a protein epitope mimetic or combinations thereof. A candidate (polypeptide) ligand may include an antibody with a mutated or chemically modified amino acid Fc region, e.g., which prevents or decreases ADCC (antibody-dependent cellular cytotoxicity) activity and/or increases half-life when compared with a wild type Fc region. Candidate (polypeptide) ligands may include antibodies with different CDRs (Complementarity-Determining Regions).

The protein structure prediction system described herein can also be used to obtain a diagnostic antibody marker of a disease. There is also provided a method that, for each of one or more candidate antibodies e.g. as described above, uses the protein structure prediction system to determine a predicted structure of the candidate antibody. The method may also involve obtaining a target protein structure of a target protein, evaluating an interaction between the predicted structure of each of the one or more candidate antibodies and the target protein structure, and selecting one of the one or more of the candidate antibodies as the diagnostic antibody marker dependent on a result of the evaluating, e.g. selecting one or more candidate antibodies that have the highest affinity to the target protein structure. The method may include making the diagnostic antibody marker. The diagnostic antibody marker may be used to diagnose a disease by detecting whether it binds to the target protein in a sample obtained from a patient, e.g. a sample of bodily fluid. As described above, a corresponding technique can be used to obtain a therapeutic antibody (polypeptide ligand).

Misfolded proteins are associated with a number of diseases. Thus in a further aspect there is provided a method of using the protein structure prediction system to identify the presence of a protein mis-folding disease. The method may include obtaining an amino acid sequence of a protein and using the protein structure prediction system to determine a structure of the protein. The method may further include obtaining a structure of a version of the protein obtained from a human or animal body, e.g., by conventional (physical) methods. The method may then include comparing the structure of the protein with the structure of the version obtained from the body and identifying the presence of a protein mis-folding disease dependent upon a result of the comparison. That is, mis-folding of the version of the protein from the body may be determined by comparison with the in silico determined structure.

In general identifying the presence of a protein mis-folding disease may involve obtaining an amino acid sequence of a protein, using an amino acid sequence of the protein to determine a structure of the protein, as described herein, and comparing the structure of the protein with the structure of a baseline version of the protein, identifying the presence of a protein mis-folding disease dependent upon a result of the comparison. For example the compared structures may be those of a mutant and wild-type protein. In implementations the wild-type protein may be used as the baseline version but in principle either may be used as the baseline version.

In some other aspects a computer-implemented method as described above or herein may be used to identify active/binding/blocking sites on a target protein from its amino acid sequence.

According to a first aspect, there is provided a method performed by one or more data processing apparatus for predicting a structure of a protein comprising one or more chains, wherein each chain comprises a sequence of amino acids, the method comprising: obtaining an initial multiple sequence alignment (MSA) representation that represents a respective MSA corresponding to each chain in the protein; obtaining a respective initial pair embedding for each pair of amino acids in the protein; processing an input comprising the initial MSA representation and the initial pair embeddings using an embedding neural network to generate an output that comprises a final MSA representation and a respective final pair embedding for each pair of amino acids in the protein, wherein the embedding neural network comprises a sequence of update blocks, wherein each update block has a respective set of update block parameters and performs operations comprising: receiving a current MSA representation and a respective current pair embedding for each pair of amino acids in the protein; updating the current MSA representation, in accordance with values of the update block parameters of the update block, based on the current pair embeddings; and updating the current pair embeddings, in accordance with the values of the update block parameters of the update block, based on the updated MSA representation; and determining a predicted structure of the protein using the final MSA representation, the final pair embeddings, or both.

In some implementations, the current MSA representation comprises a plurality of embeddings, and updating the current MSA representation based on the current pair embeddings comprises: updating the current MSA representation using attention over the embeddings in the MSA representation, wherein the attention is conditioned on the current pair embeddings.

In some implementations, updating the current MSA representation using attention over the embeddings in the current MSA representation comprises: generating, based on the current MSA representation, a plurality of attention weights; generating, based on the current pair embeddings, a respective attention bias corresponding to each of the attention weights; generating a plurality of biased attention weights based on the attention weights and the attention biases; and

updating the embeddings in the current MSA representation using attention over the embeddings in the current MSA representation based on the biased attention weights.

In some implementations, updating the embeddings in the current MSA representation using attention based on the biased attention weights comprises, for each embedding in the current MSA representation: updating the embedding, based on the biased attention weights, using attention over only embeddings in the MSA representation that are located in a same row as the embedding in an arrangement of the embeddings in the current MSA representation into a two-dimensional array.

In some implementations, updating the current pair embeddings based on the updated MSA representation comprises: applying a transformation operation to the updated MSA representation; and updating the current pair embeddings by adding a result of transformation operation to the current pair embeddings.

In some implementations, the transformation operation comprises an outer product mean operation.

In some implementations, updating the current pair embeddings based on the updated MSA representation further comprises, after adding the result of the transformation operation to the current pair embeddings: updating the current pair embeddings using attention over the current pair embeddings, wherein the attention is conditioned on the current pair embeddings.

In some implementations, updating the current pair embeddings using attention over the current pair embeddings comprises: generating, based on the current pair embeddings, a plurality of attention weights; generating, based on the current pair embeddings, a respective attention bias corresponding to each attention weight; generating a plurality of biased attention weights based on the attention weights and the attention biases; and updating the current pair embeddings using attention over the current pair embeddings based on the biased attention weights.

In some implementations, updating the current pair embeddings using attention over the current pair embeddings based on the biased attention weights comprises, for each current pair embedding: updating the current pair embedding, based on the biased attention weights, using attention over only current pair embeddings that are located in a same row as the current pair embedding in an arrangement of the current pair embeddings into a two-dimensional array.

In some implementations, updating the current pair embeddings using attention over the current pair embeddings based on the biased attention weights comprises, for each current pair embedding: updating the current pair embedding, based on the biased attention weights, using attention over only current pair embeddings that are located in a same column as the current pair embedding in an arrangement of the current pair embeddings into a two-dimensional array.

In some implementations, the protein comprises a plurality of chains, and obtaining the initial MSA representation that represents a respective multiple MSA corresponding to each chain in the protein comprises: obtaining a respective representation of the MSA corresponding to each chain in the protein as a two-dimensional array of embeddings; and assembling the representations of the MSAs corresponding to the chains into a block diagonal array.

In some implementations, determining the predicted structure of the protein comprises processing an input comprising the final pair embeddings using a folding neural network to generate an output that defines the predicted structure of the protein, comprising, for each of a plurality of pairs of amino acids in the protein: processing a final pair embedding for the pair of amino acids, in accordance with values of folding neural network parameters, to generate an output specifying a probability distribution over a set of possible distances between the pair of amino acids in a structure of the protein.

In some implementations, determining the predicted structure of the protein comprises processing an input comprising the final pair embeddings using a folding neural network to generate an output that defines the predicted structure of the protein, comprising: obtaining an initial single embedding and initial values of structure parameters for each amino acid in the protein, wherein the structure parameters for each amino acid comprise location parameters that specify a predicted three-dimensional spatial location of the amino acid in the structure of the protein; processing a folding network input comprising the final pair embeddings, the initial embedding for each amino acid in the protein, and the initial values of the structure parameters for each amino acid in the protein using the folding neural network to generate a network output comprising final values of the structure parameters for each amino acid in the protein, wherein the folding neural network comprises a plurality of update blocks, wherein each update block comprises a plurality of neural network layers and is configured to: receive an update block input comprising the final pair embeddings, a current single embedding for each amino acid in the protein, and current values of the structure parameters for each amino acid in the protein; and process the update block input to update the current single embedding and the current values of the structure parameters for each amino acid in the protein; wherein the final values of the structure parameters for each amino acid in the amino acid sequence collectively characterize the predicted structure of the protein.

In some implementations, obtaining the initial single embedding for each amino acid in the protein comprises: determining the initial single embedding for each amino acid in the protein based on the final MSA representation.

In some implementations, the structure parameters for each amino acid further comprise rotation parameters that specify a predicted spatial orientation of the amino acid in the structure of the protein.

In some implementations, the rotation parameters define a $3 \times 3$ rotation matrix.

In some implementations, processing the update block input to update the current single embedding and the current values of the structure parameters for each amino acid in the protein comprises: updating the current single embedding for each amino acid in the protein; and updating the current values of the structure parameters for each amino acid in the protein based on the updated single embeddings for the amino acids in the protein.

In some implementations, a final update block of the plurality of update blocks is further configured to generate an output that defines a predicted three-dimensional spatial location of each atom in each amino acid in the protein.

In some implementations, generating an output that defines a respective three-dimensional spatial location of each atom in each amino acid in the protein comprises, for each amino acid: processing the updated single embedding for the amino acid to generate a respective value of each of a plurality of torsion angles of bonds between the atoms in the amino acid; and determining a spatial location of each atom in the amino acid in a local reference frame of the amino acid based on the values of the plurality of torsion angles.

In some implementations, the method further comprises, for each amino acid, determining a spatial location of each atom in the amino acid in a global reference frame of the protein based on: (i) the spatial locations of the atoms in the local reference frame of the amino acid, and (ii) the updated values of the structure parameters for the amino acid.

In some implementations, updating the current single embedding for a target amino acid in the protein comprises: determining a respective attention weight between the target amino acid and each source amino acid in the protein, comprising: generating a three-dimensional query embedding of the target amino acid based on the current single embedding of the target amino acid; generating, for each source amino acid in the protein, a three-dimensional key embedding of the source amino acid based on the current single embedding of the source amino acid; and determining the attention weight between the target amino acid and each source amino acid in the protein based at least in part on a difference between: (i) the three-dimensional query embedding of the target amino acid, and (ii) the three-dimensional key embedding of the source amino acid; and updating the current single embedding of the target amino acid using the attention weights.

In some implementations, generating the three-dimensional query embedding of the target amino acid based on the current single embedding of the target amino acid comprises: processing the current single embedding of the target amino acid using a linear neural network layer that generates a three-dimensional output; and applying a rotation operation and a translation operation to the three-dimensional output of the linear neural network layer, wherein the rotation operation is specified by the current values of the rotation parameters for the target amino acid and the translation operation is specified by the current values of the location parameters for the target amino acid.

In some implementations, generating a three-dimensional key embedding of the source amino acid based on the current single embedding of the source amino acid comprises: processing the current single embedding of the source amino acid using a linear neural network layer that generates a three-dimensional output; and applying a rotation operation and a translation operation to the three-dimensional output of the linear neural network layer, wherein the rotation operation is specified by the current values of the rotation parameters for the source amino acid and the translation operation is specified by the current values of the location parameters for the source amino acid.

In some implementations, determining the attention weight between the target amino acid and each source amino acid in the protein further comprises, for each source amino acid in the protein: determining a projection of a final pair embedding corresponding to a pair of amino acids that comprises the target amino acid and the source amino acid; and determining the attention weight between the target amino acid and the source amino acid based at least in part on the projection of the final pair embedding corresponding to the pair of amino acids that comprises the target amino acid and the source amino acid.

In some implementations, updating the current single embedding of the target amino acid using the attention weights comprises: generating, for each amino acid in the protein, a three-dimensional value embedding of the amino acid based on the current single embedding of the amino acid; determining a weighted linear combination of the three-dimensional value embeddings of the amino acids using the attention weights; generating a geometric return embedding by applying a rotation operation and a translation operation to the weighted linear combination, wherein the rotation operation inverts a rotation operation specified by the current values of the rotation parameters for the target amino acid and the translation operation is specified by a negative of the current values of the location parameters for the target amino acid; and updating the current embedding of the target amino acid based on the geometric return embedding.

In some implementations, updating the current values of the structure parameters for each amino acid in the protein based on the updated single embeddings for the amino acids in the protein comprises, for each amino acid: determining updated values of the location parameters for the amino acid as a sum of: (i) the current values of the location parameters for the amino acid, and (ii) a linear projection of the updated single embedding of the amino acid; determining updated values of the rotation parameters for the amino acid as a composition of: (i) a rotation operation specified by the current values of the rotation parameters for the amino acid, and (ii) a rotation operation specified by a quaternion with real part 1 and imaginary part specified by a linear projection of the updated single embedding of the amino acid.

In some implementations, the method further comprises, for each amino acid in the protein:
processing the updated current single embedding for the amino acid that is generated by a final update block to generate a confidence estimate for a position of an atom in the amino acid in the predicted structure of the protein, wherein the confidence estimate defines a probability distribution over a range of possible accuracies for the position of the atom.

Particular embodiments of the subject matter described in this specification can be implemented so as to realize one or more of the following advantages.

The system described in this specification predicts the structure of a protein using both: (i) a multiple sequence alignment (MSA) representation that represents a respective MSA corresponding to each chain in the protein, and (2) a respective pair embedding for each pair of amino acids in the protein.

Generally, the MSA representation and the pair embeddings can encode complementary information. For example, the MSA representation can encode information about the correlations between the identities of the amino acids in different positions among a set of evolutionarily-related amino acid chains, and the pair embeddings can encode information about the inter-relationships between the amino acids in the protein.

To predict the protein structure, the system repeatedly updates the MSA representation and the pair embeddings. In particular, the system alternates between updating the MSA representation using the pair embeddings and updating the pair embeddings using the MSA representation. Updating the MSA representation using the pair embeddings enriches the information content of the MSA representation using the complementary information encoded in the pair embeddings. Conversely, updating the pair embeddings using the MSA representation enriches the information content of the pair embeddings using the complementary information encoded in the MSA representation. As a result of this enrichment, the MSA representation and the pair embeddings encode information that is more relevant to predicting the protein structure, and the system can use the MSA representation and the pair embeddings to predict the protein structure more accurately.

The system described in this specification can predict the structure of a protein by a single forward pass through a collection of jointly trained neural networks, which may take less than one second. In contrast, some conventional systems predict the structure of a protein by an extended search process through the space of possible protein structures to optimize a scalar score function, e.g., using simulated annealing or gradient descent techniques. Such a search process may require millions of search iterations and consume hundreds of central processing unit (CPU) hours. Predicting protein structures by a single forward pass through a collection of neural networks may enable the system described in this specification to consume fewer computational resources (e.g., memory and computing power) than systems that predict protein structures by an iterative search process.

The structure of a protein determines the biological function of the protein. Therefore, determining protein structures may facilitate understanding life processes (e.g., including the mechanisms of many diseases) and designing proteins (e.g., as drugs, or as enzymes for industrial processes). For example, which molecules (e.g., drugs) will bind to a protein (and where the binding will occur) depends on the structure of the protein. Since the effectiveness of drugs can be influenced by the degree to which they bind to proteins (e.g., in the blood), determining the structures of different proteins may be an important aspect of drug development. However, determining protein structures using physical experiments (e.g., by x-ray crystallography) can be time-consuming and very expensive. Therefore, the protein prediction system described in this specification may facilitate areas of biochemical research and engineering which involve proteins (e.g.,

drug development).

The details of one or more embodiments of the subject matter of this specification are set forth in the accompanying drawings and the description below. Other features, aspects, and advantages of the subject matter will become apparent from the description, the drawings, and the claims.

BRIEF DESCRIPTION OF THE DRAWINGS

FIG. 1 shows an example protein structure prediction system.

FIG. 2 shows an example architecture of an embedding neural network.

FIG. 3 shows an example architecture of an update block of the embedding neural network.

FIG. 4 shows an example architecture of a MSA update block.

FIG. 5 shows an example architecture of a pair update block.

FIG. 6 shows an example architecture of a folding neural network.

FIG. 7 illustrates the torsion angles between the bonds in the amino acid.

FIG. 8 is an illustration of an unfolded protein and a folded protein.

FIG. 9 is a flow diagram of an example process for predicting the structure of a protein.

FIG. 10 shows an example process for generating a MSA representation for an amino acid chain in a protein.

FIG. 11 shows an example process for generating a respective pair embedding for each pair of amino acids in a protein.

Like reference numbers and designations in the various drawings indicate like elements.

DETAILED DESCRIPTION

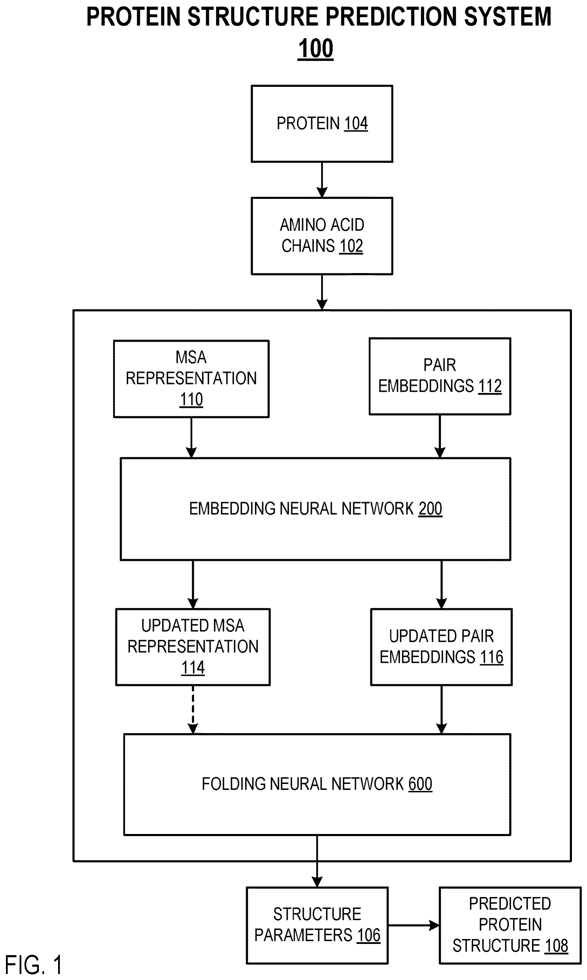

FIG. 1 shows an example protein structure prediction system 100. The protein structure prediction system 100 is an example of a system implemented as computer programs on one or more computers in one or more locations in which the systems, components, and techniques described below are implemented.

The system 100 is configured to process data defining one or more amino acid chains 102 of a protein 104 to generate a set of structure parameters 106 that define a predicted protein structure 108, i.e., a prediction of the structure of the protein 104. That is, the predicted structure 108 of the protein 104 can be defined by a set of structure parameters 106 that collectively define a predicted three-dimensional structure of the protein after the protein undergoes protein folding.

The structure parameters 106 defining the predicted protein structure 108 may be as previously described. For example they may include, e.g., location parameters and rotation parameters for each amino acid in the protein 104, a distance map that characterizes estimated distances between each pair of amino acids in the protein, a respective spatial location of each atom or backbone atom in each amino acid in the structure of the protein, or a combination thereof, as described above.

To generate the structure parameters 106 defining the predicted protein structure 108, the system 100 generates: (i) a multiple sequence alignment (MSA) representation 110 for the protein, and (ii) a set of "pair" embeddings 112 for the protein, as will be described in more detail next.

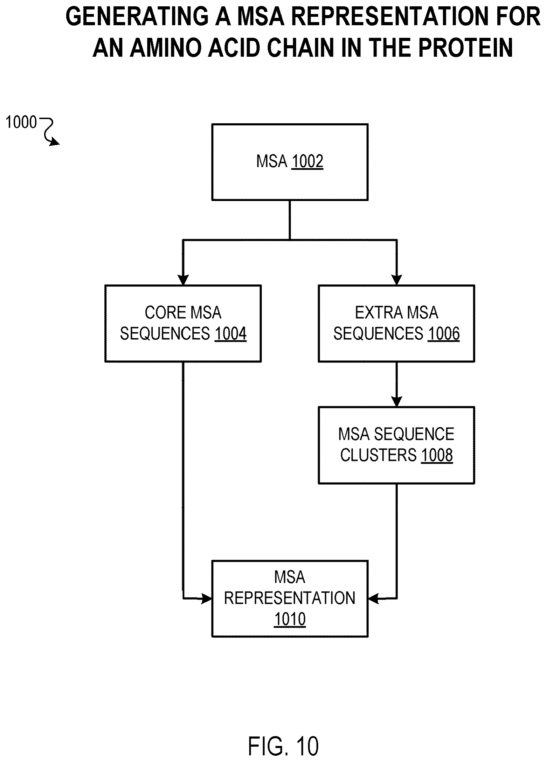

The MSA representation 110 for the protein includes a respective representation of a MSA for each amino acid chain in the protein. A MSA representation for an amino acid chain in the protein can be represented as a $M \times N$ array of embeddings (i.e., a 2-D array of embeddings having M rows and $N$ columns), where $N$ is the number of amino acids in the amino acid chain. Each row of the MSA representation can correspond to a respective MSA sequence for the amino acid chain in the protein. An example process for generating a MSA representation for an amino acid chain in the protein is described with reference to FIG. 10.

The system 100 generates the MSA representation 110 for the protein 104 from the MSA representations for the amino acid chains in the protein.

If the protein includes only a single amino acid chain, then the system 100 can identify the MSA representation 110 for the protein 104 as being the MSA representation for the single amino acid chain in the protein.

If the protein includes multiple amino acid chains, then the system 100 can generate the MSA representation 110 for the protein by assembling the MSA representations for the amino acid chains in the protein into a block diagonal 2-D array of embeddings, i.e., where the MSA representations for the amino acid chains in the protein form the blocks on the diagonal. The system 100 can initialize the embeddings at each position in the 2-D array outside the blocks on the diagonal to be a default embedding, e.g., a vector of zeros. The amino acid chains in the protein can be assigned an arbitrary ordering, and the MSA representations of the amino acid chains in the protein can be ordered accordingly in the block diagonal matrix. For example, the MSA representation for the first amino acid chain (i.e., according to the ordering) can be the first block on the diagonal, the MSA representation for the second amino acid chain can be the second block on the diagonal, and so on.

Generally, the MSA representation 110 for the protein can be represented as a 2-D array of embeddings. Throughout this specification, a "row" of the MSA representation for the protein refers to a row of a 2-D array of embeddings defining the MSA representation for the protein. Similarly, a "column" of the MSA representation for the protein refers to a column of a 2-D array of embeddings defining the MSA representation for the protein.

The set of pair embeddings 112 includes a respective pair embedding corresponding to each pair of amino acids in the protein 104. In general a pair embedding represents i.e. encodes information about the relationship between a pair of amino acids in the protein. A pair of amino acids refers to an ordered tuple that includes a first amino acid and a second amino acid in the protein, i.e., such that the set of possible pairs of amino acids in the protein is given by:

$$\{(A_i, A_j): 1 \leq i, j \leq N\} \quad (1)$$

where N is the number of amino acids in the protein, $i,j \in \{1, ..., N\}$ index the amino acids in the protein, $A_i$ is the amino acid in the protein indexed by $i$, and $A_j$ is the amino acid in the protein indexed by $j$. If the protein includes multiple amino acid chains, then the amino acids in the protein can be sequentially indexed from $\{1, ..., N\}$ in accordance with the ordering of the amino acid chains in the protein. That is, the amino acids from the first amino acid chain are sequentially indexed first, followed by the amino acids from the second amino acid chain, followed by the amino acids from the third amino acid chain, and so on. The set of pair embeddings 112 can be represented as a 2-D, $N \times N$ array of pair embeddings, e.g., where the rows of the 2-D array are indexed by $i \in \{1, ..., N\}$, the columns of the 2-D array are indexed by $j \in \{1, ..., N\}$, and position $(i,j)$ in the 2-D array is occupied by the pair embedding for the pair of amino acids $(A_i, A_j)$.

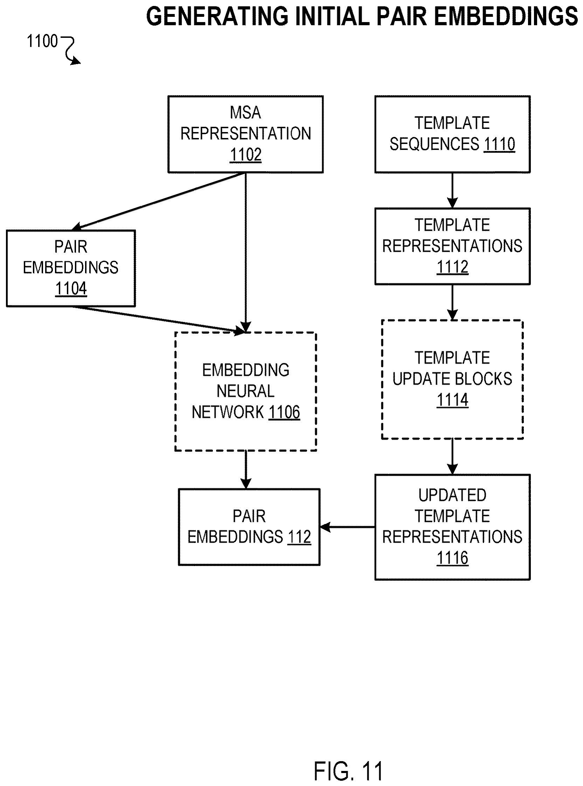

An example process for generating (initializing) a respective pair embedding corresponding to each pair of amino acids in the protein is described with reference to FIG. 11.

The system 100 generates the structure parameters 106 defining the predicted protein structure 108 using both the MSA representation 110 and the pair embeddings 112, because both have complementary properties. The structure of the MSA representation 110 can explicitly depend on the number of amino acid chains in the MSAs corresponding to each amino acid chain in the protein. Therefore, the MSA representation 110 may be inappropriate for use in directly predicting the protein structure, because the protein structure 108 has no explicit dependence on the number of amino acids chains in the MSAs. In contrast, the pair embeddings 112 characterize relationships between respective pairs of amino acids in the protein 104 and are expressed without explicit reference to the MSAs, and are therefore a convenient and effective data representation for use in predicting the protein structure 108.

The system 100 processes the MSA representation 110 and the pair embeddings 112 using an embedding neural network 200, in accordance with the values of a set of parameters of the embedding neural network 200, to update the MSA representation 110 and the pair embeddings 112. That is, the embedding neural network 200 processes the MSA representation 110 and the pair embeddings 112 to generate an updated MSA representation 114 and updated pair embeddings 116.

The embedding neural network 200 updates the MSA representation 110 and the pair embeddings 112 by sharing information between the MSA representation 110 and the pair embeddings 112. More specifically, the embedding neural network 200 alternates between updating the current MSA representation 110 based on the current pair embeddings 112, and updating the current pair embeddings 112 based on the current MSA representation 110.

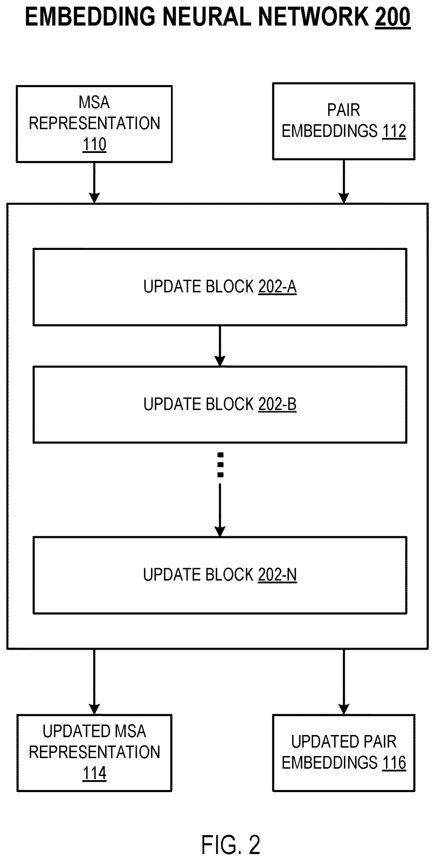

An example architecture of the embedding neural network 200 is described in more detail with reference to FIG. 2.

The system 100 generates a network input for a folding neural network 600 from the updated pair embeddings 116, the updated MSA representation 114, or both, and processes the network input using the folding neural network 600 to generate the structure parameters 106 defining the predicted protein structure.

In some implementations, the folding neural network 600 processes the updated pair embeddings 116 to generate a distance map that includes, for each pair of amino acids in the protein, a probability distribution over a set of possible distances between the pair of amino acids in the protein structure. For example, to generate the probability distribution over the set of possible distances between a pair of amino acids in the protein structure, the folding neural network may apply one or more fully-connected neural network layers to an updated pair embedding 116 corresponding to the pair of amino acids.

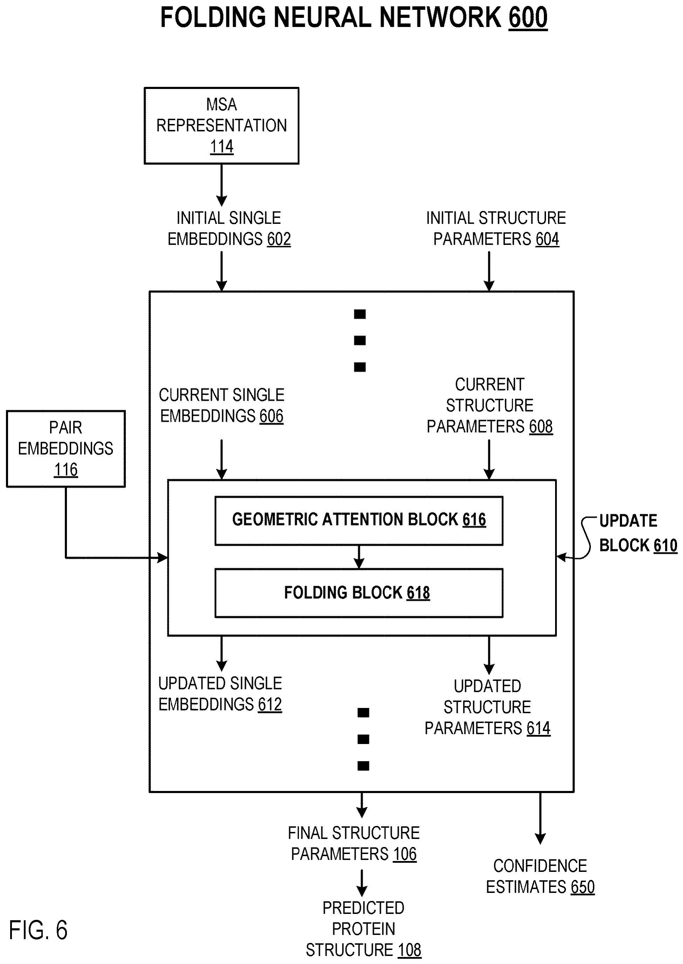

In some implementations, the folding neural network 600 generates the structure parameters 106 by processing a network input derived from both the updated MSA representation 114 and the updated pair embeddings 116 using a geometric attention operation that explicitly reasons about the 3-D geometry of the amino acids in the protein structure. An example architecture of the folding neural network 600 that implements a geometric attention mechanism is described with reference to FIG. 6.

A training engine may train the protein structure prediction system 100 from end-to-end to optimize an objective function referred to herein as a structure loss. The training engine may train the system 100 on a set of training data including multiple training examples. Each training example may specify: (i) a training input that includes an initial MSA representation and initial pair embeddings for a protein, and (ii) a target protein structure that should be generated by the system 100 by processing the training input. Target protein structures used for training the system 100 may be determined using experimental techniques, e.g., x-ray crystallography.

The structure loss may characterize a similarity between: (i) a predicted protein structure generated by the system 100, and (ii) the target protein structure that should have been generated by the system.

For example, if the predicted structure parameters define predicted location parameters and predicted rotation parameters for each amino acid in the protein, then the structure loss $\mathcal{L}_{structure}$ may be given by:

$$\mathcal{L}_{structure} = \frac{1}{N^2} \sum_{i,j=1}^{N} \left( 1 - \frac{|t_{ij} - \widetilde{t_{ij}}|}{A} \right)_+ \quad (2)$$

$$t_{ij} = R_i^{-1}(t_j - t_i) \quad (3)$$

$$\widetilde{t_{ij}} = \widetilde{R}_i^{-1}(\widetilde{t}_j - \widetilde{t}_i) \quad (4)$$

where $N$ is the number of amino acids in the protein, $t_i$ denote the predicted location parameters for amino acid $i$, $R_i$ denotes a $3 \times 3$ rotation matrix specified by the predicted rotation parameters for amino acid $i$, $\widetilde{t}_i$ are the target location parameters for amino acid $i$, $\widetilde{R}_i$ denotes a $3 \times 3$ rotation matrix specified by the target rotation parameters for amino acid $i$, $A$ is a constant, $R_i^{-1}$ refers to the inverse of the $3 \times 3$ rotation matrix specified by predicted rotation parameters $R_i$, $\widetilde{R}_i^{-1}$ refers to the inverse of the $3 \times 3$ rotation matrix specified by the target rotation parameters $\widetilde{R}_i$, and $(\cdot)_+$ denotes a rectified linear unit (ReLU) operation.

The structure loss defined with reference to equations (2)-(4) may be understood as averaging the loss $|t_{ij} - \widetilde{t_{ij}}|$ over each pair of amino acids in the protein. The term $t_{ij}$ defines the predicted spatial location of amino acid j in the predicted frame of reference of amino acid i, and $\widetilde{t_{ij}}$ defines the actual spatial location of amino acid $j$ in the actual frame of reference of amino acid i. These terms are sensitive to the predicted and actual rotations of amino acid i and $j$, and therefore carry richer information than loss terms that are only sensitive to the predicted and actual distances between amino acids.

As another example, if the predicted structure parameters define predicted spatial locations of each atom in each amino acid of the protein, then the structure loss may be an average error (e.g., squared-error) between: (i) the predicted spatial locations of the atoms, and (ii) the target (e.g., ground truth) spatial locations of the atoms.

Optimizing the structure loss encourages the system 100 to generate predicted protein structures that accurately approximate true protein structures.

In addition to optimizing the structure loss, the training engine may train the system 100 to optimize one or more auxiliary losses. The auxiliary losses may penalize predicted structures having characteristics that are unlikely to occur in the natural world, e.g., based on the bond angles and/or bond lengths of the bonds between the atoms in the amino acids in the predicted structures, or based on the proximity of the atoms in different amino acids in the predicted structures.

The training engine may train the structure prediction system 100 on the training data over multiple training iterations, e.g., using stochastic gradient descent training techniques.

FIG. 2 shows an example architecture of an embedding neural network 200 that is configured to process the MSA representation 110 and the pair embeddings 112 to generate the updated MSA representation 114 and the updated pair embeddings 116.

The embedding neural network 200 includes a sequence of update blocks 202-A-N. Throughout this specification, a "block" refers to a portion of a neural network, e.g., a subnetwork of the neural network that includes one or more neural network layers.

Each update block in the embedding neural network is configured to receive a block input that includes a MSA representation and a pair embedding, and to process the block input to generate a block output that includes an updated MSA representation and an updated pair embedding.

The embedding neural network 200 provides the MSA representation 110 and the pair embeddings 112 included in the network input of the embedding neural network 200 to the first update block (i.e., in the sequence of update blocks). The first update block processes the MSA representation 110 and the pair embeddings 112 to generate an updated MSA representation and updated pair embeddings.

For each update block after the first update block, the embedding neural network 200 provides the update block with the MSA representation and the pair embeddings generated by the preceding update block, and provides the updated MSA representation and the updated pair embeddings generated by the update block to the next update block.

The embedding neural network 200 gradually enriches the information content of the MSA representation 110 and the pair embeddings 112 by repeatedly updating them using the sequence of update blocks 202-A-N.

The embedding neural network 200 may provide the updated MSA representation 114 and the updated pair embeddings 116 generated by the final update block (i.e., in the sequence of update blocks) as the network output.

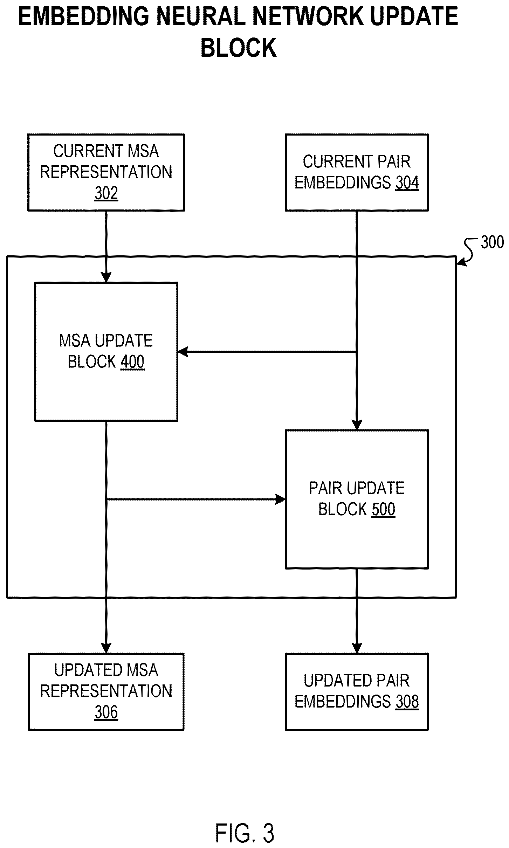

FIG. 3 shows an example architecture of an update block 300 of the embedding neural network 200, i.e., as described with reference to FIG. 2.

The update block 300 receives a block input that includes the current MSA representation 302 and the current pair embeddings 304, and processes the block input to generate the updated MSA representation 306 and the updated pair embeddings 308.

The update block 300 includes an MSA update block 400 and a pair update block 500.

The MSA update block 400 updates the current MSA representation 302 using the current pair embeddings 304, and the pair update block 500 updates the current pair embeddings 304 using the updated MSA representation 306 (i.e., that is generated by the MSA update block 400).

Generally, the MSA representation and the pair embeddings can encode complementary information. For example, the MSA representation can encode information about the correlations between the identities of the amino acids in different positions among a set of evolutionarily-related amino acid chains, and the pair embeddings can encode information about the inter-relationships between the amino acids in the protein. The MSA update block 400 enriches the information content of the MSA representation using complementary information encoded in the pair embeddings, and the pair update block 500 enriches the information content of the pair embeddings using complementary information encoded in the MSA representation. As a result of this enrichment, the updated MSA representation and the updated pair embedding encode information that is more relevant to predicting the protein structure.

The update block 300 is described herein as first updating the current MSA representation 302 using the current pair embeddings 304, and then updating the current pair embeddings 304 using the updated MSA representation 306. The description should not be understood as limiting the update block to performing operations in this sequence, e.g., the update block could first update the current pair embeddings using the current MSA representation, and then update the current MSA representation using the updated pair embeddings.

The update block 300 is described herein as including an MSA update block 400 (i.e., that updates the current MSA representation) and a pair update block 500 (i.e., that updates the current pair embeddings). The description should not be understood to limiting the update block 300 to include only one MSA update block or only one pair update block. For example, the update block 300 can include multiple MSA update blocks that update the MSA representation multiple times before the MSA representation is provided to a pair update block for use in updating the current pair embeddings. As another example, the update block 300 can include multiple pair update blocks that update the pair embeddings multiple times using the MSA representation.

The MSA update block 400 and the pair update block 500 can have any appropriate architectures that enable them to perform their described functions.

In some implementations, the MSA update block 400, the pair update block 500, or both, include one or more "self-attention" blocks. As used throughout this document, a self-attention block generally refers to a neural network block that updates a collection of embeddings, i.e., that receives a collection of embeddings and outputs updated embeddings. To update a given embedding, the self-attention block can determine a respective "attention weight" between the given embedding and each of one or more selected embeddings, and then update the given embedding using: (i) the attention weights, and (ii) the selected embeddings. For convenience, the self-attention block may be said to update the given embedding using attention "over" the selected embeddings.

For example, a self-attention block may receive a collection of input embeddings $\{x_i\}_{i=1}^{N}$, where $N$ is the number of amino acids in the protein, and to update embedding $x_i$, the self-attention block may determine attention weights $\left[a_{i,j}\right]_{j=1}^{N}$ where $a_{i,j}$ denotes the attention weight between $x_i$ and $x_j$, as:

$$\left[a_{i,j}\right]_{j=1}^{N} = \text{softmax}\left(\frac{\left(W_q x_i\right)K^T}{c}\right) \quad (5)$$

$$K^T = \left[W_k x_j\right]_{j=1}^{N} \quad (6)$$

where $W_q$ and $W_k$ are learned parameter matrices, softmax($\cdot$) denotes a soft-max normalization operation, and $c$ is a constant. Using the attention weights, the self-attention layer may update embedding $x_i$ as:

$$x_i \leftarrow \sum_{j=1..N} a_{i,j} \cdot \left(W_v x_j\right) \quad (7)$$

where $W_v$ is a learned parameter matrix. ($W_q x_i$ can be referred to as the "query embedding" for input embedding $x_i$, $W_k x_j$ can be referred to as the "key embedding" for input embedding $x_i$, and $W_v x_j$ can be referred to as the "value embedding" for input embedding $x_i$).

The parameter matrices $W_q$ (the "query embedding matrix"), $W_k$ (the "key embedding matrix"), and $W_v$ (the "value embedding matrix") are trainable parameters of the self-attention block. The parameters of any self-attention blocks included in the MSA update block 400 and the pair update block 500 can be understood as being parameters of the update block 300 that can be trained as part of the end-to-end training of the protein structure prediction system 100 described with reference to FIG. 1. Generally, the (trained) parameters of the query, key, and value embedding matrices are different for different self-attention blocks, e.g., such that a self-attention block included in the MSA update block 400 can have different query, key, and value embedding matrices with different parameters than a self-attention block included in the pair update block 500.

In some implementations, the MSA update block 400, the pair update block 500, or both, include one or more self-attention blocks that are conditioned on the pair embeddings, i.e., that implement self-attention operations that are conditioned on the pair embeddings. To condition a self-attention operation on the pair embeddings, the self-attention block can process the pair embeddings to generate a respective "attention bias" corresponding to each attention weight. For example, in addition to determining the attention weights $\left[a_{i,j}\right]_{j=1}^{N}$ in accordance with equations (5)-(6), the self-attention block can generate a corresponding set of attention biases $\left[b_{i,j}\right]_{j=1}^{N}$, where $b_{i,j}$ denotes the attention bias between $x_i$ and $x_j$. The self-attention block can generate the attention bias $b_{i,j}$ by applying a learned parameter matrix to the pair embedding $h_{i,j}$, i.e., for the pair of amino acids in the protein indexed by $(i, j)$.

The self-attention block can determine a set of "biased attention weights" $\left[c_{i,j}\right]_{j=1}^{N}$, where $c_{i,j}$ denotes the biased attention weight between $x_i$ and $x_j$, e.g., by summing (or otherwise combining) the attention weights and the attention biases. For example, the self-attention block can determine the biased attention weight $c_{i,j}$ between embeddings $x_i$ and $x_j$ as:

$$c_{i,j} = a_{i,j} + b_{i,j}$$

where $a_{i,j}$ is the attention weight between $x_i$ and $x_j$ and $b_{i,j}$ is the attention bias between $x_i$ and $x_j$. The self-attention block can update each input embedding $x_i$ using the biased attention weights, e.g.:

$$x_i \leftarrow \sum_{j=1..N} c_{i,j} \cdot (W_v x_j) \qquad (8)$$

where $W_v$ is a learned parameter matrix.

Generally, the pair embeddings encode information characterizing the structure of the protein and the relationships between the pairs of amino acids in the structure of the protein. Applying a self-attention operation that is conditioned on the pair embeddings to a set of input embeddings allows the input embeddings to be updated in a manner that is informed by the protein structural information encoded in the pair embeddings. The update blocks of the embedding neural network can use the self-attention blocks that are conditioned on the pair embeddings to update and enrich the MSA representation and the pair embeddings themselves.

Optionally, a self-attention block can have multiple "heads" that each generate a respective updated embedding corresponding to each input embedding, i.e., such that each input embedding is associated with multiple updated embeddings. For example, each head may generate updated embeddings in accordance with different values of the parameter matrices $W_q$, $W_k$, and $W_v$ that are described with reference to equations (5)-(7). A self-attention block with multiple heads can implement a "gating" operation to combine the updated embeddings generated by the heads for an input embedding, i.e., to generate a single updated embedding corresponding to each input embedding. For example, the self-attention block can process the input embeddings using one or more neural network layers (e.g., fully connected neural network layers) to generate a respective gating value for each head. The self-attention block can then combine the updated embeddings corresponding to an input embedding in accordance with the gating values. For example, the self-attention block can generate the updated embedding for an input embedding $x_i$ as:

$$\sum_{k=1}^{K} \alpha_k \cdot x_i^{next} \qquad (9)$$

where $k$ indexes the heads, $\alpha_k$ is the gating value for head $k$, and $x_i^{next}$ is the updated embedding generated by head $k$ for input embedding $x_i$.

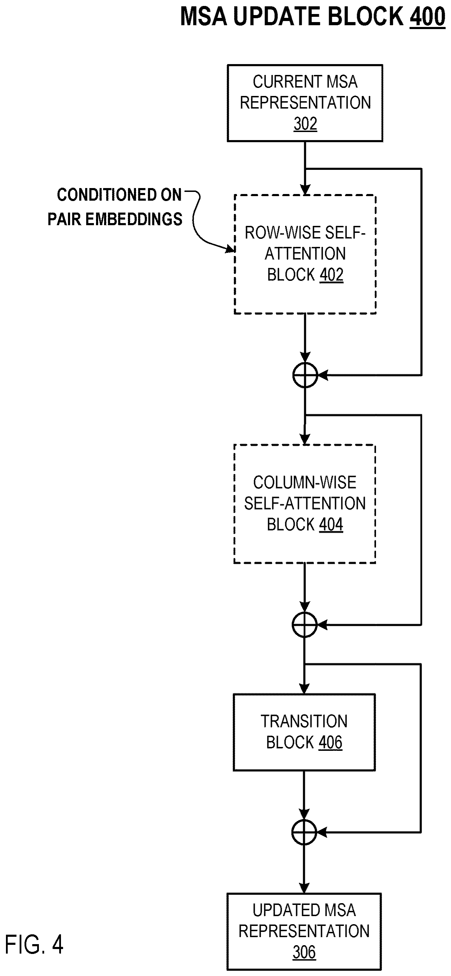

An example architecture of a MSA update block 400 that uses self-attention blocks conditioned on the pair embeddings is described with reference to FIG. 4. The example MSA update block described with reference to FIG. 4 updates the current MSA representation based on the current pair embeddings by processing the rows of the current MSA representation using a self-attention block that is conditioned on the current pair embeddings.

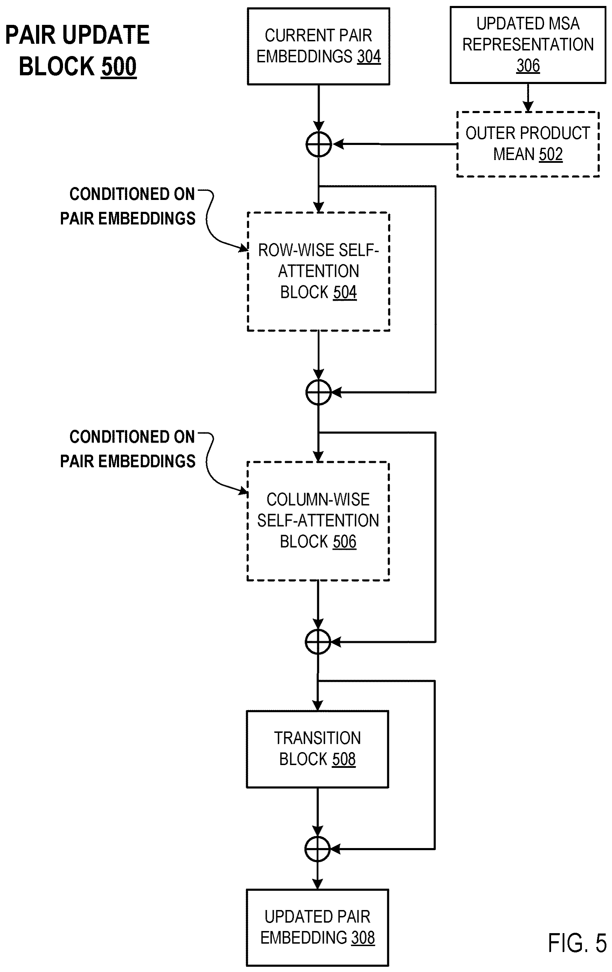

An example architecture of a pair update block 500 that uses self-attention blocks conditioned on the pair embeddings is described with reference to FIG. 5. The example pair update block described with reference to FIG. 5 updates the current pair embeddings based on the updated MSA representation by computing an outer product mean of the updated MSA representation, adding the result of the outer product mean to the current pair embeddings, and processing the current pair embeddings using self-attention blocks that are conditioned on the current pair embeddings.

FIG. 4 shows an example architecture of a MSA update block 400. The MSA update block 400 is configured to receive the current MSA representation 302, to update the current MSA representation 306 based (at least in part) on the current pair embedding.

To update the current MSA representation 302, the MSA update block 400 updates the embeddings in each row of the current MSA representation using a self-attention operation (i.e., a "row-wise" self-attention operation) that is conditioned on the current pair embeddings. More specifically, the MSA update block 400 provides the embeddings in each row of the current MSA representation 302 to a "row-wise" self-attention block 402 that is conditioned on the current pair embeddings, e.g., as described with reference to FIG. 3, to generate updated embeddings for each row of the current MSA representation 302. Optionally, the MSA update block can add the input to the row-wise self-attention block 402 to the output of the row-wise self-attention block 402. Conditioning the row-wise self-attention block 402 on the current pair embeddings enables the MSA update block 400 to enrich the current MSA representation 302 using information from the current pair embeddings.

The MSA update block then updates the embeddings in each column of the current MSA representation using a self-attention operation (i.e., a "column-wise" self-attention operation) that is not conditioned on the current pair embeddings. More specifically, the MSA update block 400 provides the embeddings in each column of the current MSA representation 302 to a "column-wise" self-attention block 404 that is not conditioned on the current pair embeddings to generate updated embeddings for each column of the current MSA representation 302. As a result of not being conditioned on the current pair embeddings, the column-wise self-attention block 404 generates updated embeddings for each column of the current MSA representation using attention weights (e.g., as described with reference to equations (5)-(6)) rather than biased attention weights (e.g., as described with reference to equation (8)). Optionally, the MSA update block can add the input to the column-wise self-attention block 404 to the output of the column-wise self-attention block 404.

The MSA update block then processes the current MSA representation 302 using a transition block, e.g., that applies one or more fully-connected neural network layers to the current MSA representation 302. Optionally, the MSA update block 400 can add the input to the transition block 406 to the output of the transition block 406.

The MSA update block can output the updated MSA representation 306 resulting from the operations performed by the row-wise self-attention block 402, the column-wise self-attention block 404, and the transition block 406.

FIG. 5 shows an example architecture of a pair update block 500. The pair update block 500 is configured to receive the current pair embeddings 304, and to update the current pair embeddings 304 based (at least in part) on the updated MSA representation 306.

To update the current pair embeddings 304, the pair update block 500 applies an outer product mean operation 502 to the updated MSA representation 306 and adds the result of the outer-product mean operation 502 to the current pair embeddings 304.

The outer product mean operation defines a sequence of operations that, when applied to an MSA representation represented as an $M \times N$ array of embeddings, generates an $N \times N$ array of embeddings, i.e, where N is the number of amino acids in the protein. The current pair embeddings 304 can also be represented as an $N \times N$ array of embeddings, and adding the result of the outer product mean 502 to the current pair embeddings 304 refers to summing the two $N \times N$ arrays of embeddings.

To compute the outer product mean, the pair update block generates a tensor $A(\cdot)$, e.g., given by:

$$A(res1, res2, ch1, ch2)$$

$$= \frac{1}{|\text{rows}|} \sum_{rows} LeftAct(row, res1, ch1) \cdot RightAct(row, res2, ch2)$$

where $res1$, $res2 \in \{1, ..., N\}$, $ch1$, $ch2 \in \{1, ..., C\}$, where C is the number of channels in each embedding of the MSA representation, |rows| is the number rows in the MSA representation, $LeftAct(row, res1, ch1)$ is a linear operation (e.g., defined by a matrix multiplication) applied to the channel $ch1$ of the embedding of the MSA representation located at the row indexed by "row" and the column indexed by "res1", and $RightAct(row, res2, ch2)$ is a linear operation (e.g., defined by a matrix multiplication) applied to the channel $ch2$ of the embedding of the MSA representation located at the row indexed by "row" and the column indexed by "res2". The result of the outer product mean is generated by flattening and linearly projecting the ($ch1$, $ch2$) dimensions of the tensor $A$. Optionally, the pair update block can perform one or more Layer Normalization operations (e.g., as described with reference to Jimmy Lei Ba et al., "Layer Normalization," ar-Xiv:1607.06450) as part of computing the outer product mean.

Generally, the updated MSA representation 306 encodes information about the correlations between the identities of the amino acids in different positions among a set of evolutionarily-related amino acid chains. The information encoded in the updated MSA representation 306 is relevant to predicting the structure of the protein, and by incorporating the information encoded in the updated MSA representation into the current pair embeddings (i.e., by way of the outer product mean 502), the pair update block 500 can enhance the information content of the current pair embeddings.

After updating the current pair embeddings 304 using the updated MSA representation (i.e., by way of the outer product mean 502), the pair update block 500 updates the current pair embeddings in each row of an arrangement of the current pair embeddings into an $N \times N$ array using a self-attention operation (i.e., a "row-wise" self-attention operation) that is conditioned on the current pair embeddings. More specifically, the pair update block 500 provides each row of current pair embeddings to a "row-wise" self-attention block 504 that is also conditioned on the current pair embeddings, e.g., as described with reference to FIG. 3, to generate updated pair embeddings for each row. Optionally, the pair update block can add the input to the row-wise self-attention block 504 to the output of the row-wise self-attention block 504.

The pair update block 500 then updates the current pair embeddings in each column of the $N \times N$ array of current pair embeddings using a self-attention operation (i.e., a "column-wise" self-attention operation) that is also conditioned on the current pair embeddings. More specifically, the pair update block 500 provides each column of current pair embeddings to a "column-wise" self-attention block 506 that is also conditioned on the current pair embeddings to generate updated pair embeddings for each column. Optionally, the pair update block can add the input to the column-wise self-attention block 506 to the output of the column-wise self-attention block 506.

The pair update block 500 then processes the current pair embeddings using a transition block, e.g., that applies one or more fully-connected neural network layers to the current pair embeddings. Optionally, the pair update block 500 can add the input to the transition block 508 to the output of the transition block 508.

The pair update block can output the updated pair embeddings 308 resulting from the operations performed by the row-wise self-attention block 504, the column-wise self-attention block 506, and the transition block 508.

FIG. 6 shows an example architecture of a folding neural network 600 that generates a set of structure parameters 106 that define the predicted protein structure 108. The folding neural network 600 can be included in the protein structure prediction system 100 described with reference to FIG. 1.

The folding neural network 600 generates structure parameters that can include: (i) location parameters, and (ii) rotation parameters, for each amino acid in the protein. As described earlier, the location parameters for an amino acid may specify a predicted 3-D spatial location of a specified atom in the amino acid in the structure of the protein. The rotation parameters for an amino acid may specify the predicted "orientation" of the amino acid in the structure of the protein. More specifically, the rotation parameters may specify a 3-D spatial rotation operation that, if applied to the coordinate system of the location parameters, causes the three "main chain" atoms in the amino acid to assume fixed positions relative to the rotated coordinate system.

In implementations the folding neural network 600 receives an input derived from the final MSA representation, the final pair embeddings, or both and generates final values of the structure parameters 106 that define a predicted structure of the protein. For example the folding neural network 600 may receive an input that includes: (i) a respective pair embedding 116 for each pair of amino acids in the protein, (ii) initial values of a "single" embedding 602 for each amino acid in the protein, and (iii) initial values of structure parameters 604 for each amino acid in the protein. The folding neural network 600 processes the input to generate final values of the structure parameters 106 that collectively characterize the predicted structure 108 of the protein.

The protein structure prediction system 100 can provide the folding neural network 600 with the pair embeddings generated as an output of an embedding neural network, as described with reference to FIG. 1.

The protein structure prediction system 100 can generate the initial single embeddings 602 for the amino acids from the MSA representation 114, i.e., that is generated as an output of an embedding neural network, as described with reference to FIG. 1. For example, as described above, the MSA representation 114 can be represented as a 2-D array of embeddings having a number of columns equal to the number of amino acids in the protein, where each column is associated with a respective amino acid in the protein. The protein structure prediction system 100 can generate the initial single embedding for each amino acid in the protein by summing (or otherwise combining) the embeddings from the column of the MSA representation 114 that is associated with the amino acid. As another example, the protein structure prediction system 100 can generate the initial single embeddings for the amino acids in the protein by extracting the embeddings from a row of the MSA representation 114 that corresponds to the amino acid sequence of the protein whose structure is being estimated.

The protein structure prediction system 100 may generate the initial structure parameters 604 with default values, e.g., where the location parameters for each amino acid are initialized to the origin (e.g., [0,0,0] in a Cartesian coordinate system), and the rotation parameters for each amino acid are initialized to a $3 \times 3$ identity matrix.

The folding neural network 600 can generate the final structure parameters 106 by repeatedly updating the current values of the single embeddings 606 and the structure parameters 608, i.e., starting from their initial values. More specifically, the folding neural network 600 includes a sequence of update neural network blocks 610, where each update block 610 is configured to update the current single embeddings 606 (i.e., to generate updated single embeddings 612) and to update the current structure parameters 608 (i.e., to generate updated structure parameters 614). The folding neural network 600 may include other neural network layers or blocks in addition to the update blocks, e.g., that may be interleaved with the update blocks.

Each update block 610 can include: (i) a geometric attention block 616, and (ii) a folding block 618, each of which will be described in more detail next.

The geometric attention block 616 updates the current single embeddings using a "geometric" self-attention operation that explicitly reasons about the 3-D geometry of the amino acids in the structure of the protein, i.e., as defined by the structure parameters. More specifically, to update a given single embedding, the geometric attention block 616 determines a respective attention weight between the given single embedding and each of one or more selected single embeddings, where the attention weights depend on both the current single embeddings, the current structure parameters, and the pair embeddings. The geometric attention block 616 then updates the given single embedding using: (i) the attention weights, (ii) the selected single embeddings, and (iii) the current structure parameters.

To determine the attention weights, the geometric attention block 616 processes each current single embedding to generate a corresponding "symbolic query" embedding, "symbolic key" embedding, and "symbolic value" embedding. For example, the geometric attention block 616 may generate the symbolic query embedding $q_i$, symbolic key embedding $k_i$, and symbolic value embedding $v_i$ for the single embedding $h_i$ corresponding to the i-th amino acid as:

$$q_i = \mathrm{Linear}(h_i) \qquad (10)$$

$$k_i = \text{Linear}(h_i) \quad (11)$$

$$v_i = \text{Linear}(h_i) \quad (12)$$

where Linear($\cdot$) refers to linear layers having independent learned parameter values.

The geometric attention block 616 additionally processes each current single embedding to generate a corresponding "geometric query" embedding, "geometric key" embedding, and "geometric value" embedding. The geometric query, geometric key, and geometric value embeddings for each single embedding are each 3-D points that are initially generated in the local reference frame of the corresponding amino acid, and then rotated and translated to a global reference frame using the structure parameters for the amino acid. For example, the geometric attention block 616 may generate the geometry query embedding $q_i^p$, geometric key embedding $k_i^p$, and geometric value embedding $v_i^p$ for the single embedding $h_i$ corresponding to the i-th amino acid as:

$$q_i^{\text{p}} = R_i \cdot \text{Linear}_{\text{p}}(h_i) + t_i \quad (13)$$

$$k_i^{\text{p}} = R_i \cdot \text{Linear}_{\text{p}}(h_i) + t_i \quad (14)$$

$$v_i^{\text{p}} = R_i \cdot \text{Linear}_{\text{p}}(h_i) + t_i \quad (15)$$

where $\text{Linear}_{\text{p}}(\cdot)$ refers to linear layers having independent learned parameter values that project $h_i$ to a 3-D point (the superscript p indicates that the quantity is a 3-D point), $R_i$ denotes the rotation matrix specified by the rotation parameters for the i-th amino acid, and $t_i$ denotes the location parameters for the i-th amino acid.

To update the single embedding $h_i$ corresponding to amino acid $i$, the geometric attention block 616 may generate attention weights $[a_j]_{j=1}^{N}$, where $N$ is the total number of amino acids in the protein and $a_j$ is the attention weight between amino acid $i$ and amino acid $j$, as:

$$[a_j]_{j=1}^{N} = \text{softmax}\left(\left[\frac{q_i \cdot k_j}{\sqrt{m}} + \alpha |q_i^p - k_j^p|_2^2 + (b_{i,j} \cdot w)\right]_{j=1}^{N}\right) \quad (16)$$

where $q_i$ denotes the symbolic query embedding for amino acid $i$, $k_j$ denotes the symbolic key embedding for amino acid $j$, $m$ denotes the dimensionality of $q_i$ and $k_j$, $\alpha$ denotes a learned parameter, $q_i^p$ denotes the geometric query embedding for amino acid $i$, $k_j^p$ denotes the geometry key embedding for amino acid $j$, $|\cdot|_2$ is an $L_2$ norm, and $b_{i,j}$ is the pair embedding 116 corresponding to the pair of amino acids that includes amino acid $i$ and amino acid $j$, and w is a learned weight vector (or some other learned projection operation).

Generally, the pair embedding for a pair of amino acids implicitly encodes information relating the relationship between the amino acids in the pair, e.g., the distance between the amino acids in the pair. By determining the attention weight between amino acid i and amino acid $j$ based in part on the pair embedding for amino acids $i$ and $j$, the folding neural network 600 enriches the attention weights with the information from the pair embedding and thereby improves the accuracy of the predicted folding structure.

In some implementations, the geometric attention block 616 generate multiple sets of geometric query embeddings, geometric key embeddings, and geometric value embeddings, and uses each generated set of geometric embeddings in determining the attention weights.

After generating the attention weights for the single embedding $h_i$ corresponding to amino acid i, the geometric attention block 616 uses the attention weights to update the single embedding $h_i$. In particular, the geometric attention block 616 uses the attention weights to generate a "symbolic return" embedding and a "geometric return" embedding, and then updates the single embedding using the symbolic return embedding and the geometric return embedding. The geometric attention block 124 may generate the symbolic return embedding $o_i$ for amino acid i, e.g., as:

$$o_i = \sum_j a_j v_j \quad (17)$$

where $\left[a_j\right]_{j=1}^N$ denote the attention weights (e.g., defined with reference to equation (16)) and each $v_j$ denotes the symbolic value embedding for amino acid $j$. The geometric attention block 616 may generate the geometric return embedding $o_i^{\mathrm{p}}$ for amino acid i, e.g., as:

$$o_i^{\mathrm{p}} = R_i^{-1} \cdot \left(\sum_j a_j v_j^p - t_i\right) \quad (18)$$

where the geometric return embedding $o_i^{\mathrm{p}}$ is a 3-D point, $\left[a_j\right]_{j=1}^N$ denote the attention weights (e.g., defined with reference to equation (16)), $R_i^{-1}$ is inverse of the rotation matrix specified by the rotation parameters for amino acid i, and $t_i$ are the location parameters for amino acid i. It can be appreciated that the geometric return embedding is initially generated in the global reference frame, and then rotated and translated to the local reference frame of the corresponding amino acid.

The geometric attention block 616 may update the single embedding $h_i$ for amino acid i using the corresponding symbolic return embedding $o_i$ (e.g., generated in accordance with equation (17)) and geometric return embedding $o_i^{\mathrm{p}}$ (e.g., generated in accordance with equation (18)), e.g., as:

$$h_i^{next} = \mathrm{LayerNorm}\left(h_i + \mathrm{Linear}\left(o_i, o_i^p, \left|o_i^p\right|\right)\right) \quad (19)$$

where $h_i^{next}$ is the updated single embedding for amino acid i, $|\cdot|$ is a norm, e.g., an $L_2$ norm, and LayerNorm($\cdot$) denotes a layer normalization operation, e.g., as described with reference to: J.L. Ba, J.R. Kiros, G.E. Hinton, "Layer Normalization," arXiv:1607.06450 (2016).

Updating the single embeddings 606 of the amino acids using concrete 3-D geometric embeddings, e.g., as described with reference to equations (13)-(15), enables the geometric attention block 616 to reason about 3-D geometry in updating the single embeddings. Moreover, each update block updates the single embeddings and the structure parameters in a manner that is invariant to rotations and translations of the overall protein structure. For example, applying the same global rotation and translation operation to the initial structure parameters provided to the folding neural network 600 would cause the folding neural network 600 to generate a predicted structure that is globally rotated and translated in the same way, but otherwise the same. Therefore, global rotation and translation operations applied to the initial structure parameters would not affect the accuracy of the predicted protein structure generated by the folding neural network 600 starting from the initial structure parameters. The rotational and translational invariance of the representations generated by the folding neural network 600 facilitates training, e.g., because the folding neural network 600 automatically learns to generalize across all rotations and translations of protein structures.

The updated single embeddings for the amino acids may be further transformed by one or more additional neural network layers in the geometric attention block 616, e.g., linear neural network layers, before being provided to the folding block 618.

After the geometric attention block 616 updates the current single embeddings 606 for the amino acids, the folding block 618 updates the current structure parameters 608 using the updated single embeddings 612. For example, the folding block 618 may update the current location parameters $t_i$ for amino acid i as:

$$t_i^{next} = t_i + \mathrm{Linear}(h_i^{next}) \quad (20)$$

where $t_i^{next}$ are the updated location parameters, Linear($\cdot$) denotes a linear neural network layer, and $h_i^{next}$ denotes the updated single embedding for amino acid *i*. In another example, the rotation parameters $R_i$ for amino acid i may specify a rotation matrix, and the folding block 618 may update the current rotation parameters $R_i$ as:

$$w_i = \text{Linear}(h_i^{next}) \quad (21)$$

$$R_i^{next} = R_i \cdot \text{QuaternionToRotation}(1 + w_i) \quad (22)$$

where $w_i$ is a three-dimensional vector, Linear($\cdot$) is a linear neural network layer, $h_i^{next}$ is the updated single embedding for amino acid i, 1 + $w_i$ denotes a quaternion with real part 1 and imaginary part $w_i$, and QuaternionToRotation($\cdot$) denotes an operation that transforms a quaternion into an equivalent $3 \times 3$ rotation matrix. Updating the rotation parameters using equations (21)-(22) ensures that the updated rotation parameters define a valid rotation matrix, e.g., an orthonormal matrix with determinant one.

The folding neural network 600 may provide the updated structure parameters generated by the final update block 610 as the final structure parameters 106 that define the predicted protein structure 108. The folding neural network 600 may include any appropriate number of update blocks, e.g., 5 update blocks, 25 update blocks, or 125 update blocks. Optionally, each of the update blocks of the folding neural network may share a single set of parameter values that are jointly updated during training of the folding neural network. Sharing parameter values between the update blocks 610 reduces the number of trainable parameters of the folding neural network and may therefore facilitate effective training of the folding neural network, e.g., by stabilizing the training and reducing the likelihood of overfitting.

During training, a training engine can train the parameters of the structure prediction system, including the parameters of the folding neural network 600, based on a structure loss that evaluates the accuracy of the final structure parameters 106, as described above. In some implementations, the training engine can further evaluate an auxiliary structure loss for one or more of the update blocks 610 that precede the final update block (i.e., that produces the final structure parameters). The auxiliary structure loss for an update block evaluates the accuracy of the updated structure parameters generated by the update block.

Optionally, during training, the training engine can apply a "stop gradient" operation to prevent gradients from backpropagating through certain neural network parameters of each update block, e.g., the neural network parameters used to compute the updated rotation parameters (as described in equations (21)-(22)). Applying these stop gradient operations can improve the numerical stability of the gradients computed during training.

Generally, a similarity between the predicted protein structure 108 generated by the folding neural network 600 and the corresponding ground truth protein structure can be measured, e.g., by a similarity measure that assigns a respective accuracy score to each of multiple atoms in the predicted protein structure. For example, the similarity measure can assign a respective accuracy score to each carbon alpha atom in the predicted protein structure. The accuracy score for an atom in the predicted protein structure can characterize how closely the position of the atom in the predicted protein structure conforms with the actual position of the atom in the ground truth protein structure. An example of a similarity measure that can compare the predicted protein structure to the ground truth protein structure to generate accuracy scores for the atoms in predicted protein structure is the lDDT similarity measure described with reference to: V. Mariani et al., "lDDT: a local superposition-free score for comparing protein structures and models using distance difference tests," Bioinformatics, 2013 Nov 1; 29(21) 2722-2728.

The folding neural network 600 can be configured to generate a respective confidence estimate 650 for each of one or more atoms in the predicted protein structure 108. The confidence estimate 650 for an atom in the predicted protein structure characterizes the predicted accuracy score (e.g., lDDT accuracy score) for the atom in the predicted protein structure, i.e., that would be generated by a similarity measure that compares the predicted protein structure to the (potentially unknown) ground truth protein structure. In one example, the confidence estimate 650 for an atom in the predicted protein structure can define a discrete probability distribution over a set of intervals that form a partition of a range of possible values for the accuracy score for the atom. The discrete probability distribution can associate a respective probability with each of the intervals that defines the likelihood that the actual accuracy score is included in the interval. For example, range of possible values of the accuracy score may be [0, 100], and the confidence estimate 650 may define a probability distribution over the set of intervals {[0, 2), [2, 4), ..., [98,100]}. In another example, the confidence estimate 650 for an atom in the predicted protein structure can be a numerical value, i.e., that directly predicts the accuracy score for the atom.

In some implementations, the folding neural network 600 generates a respective confidence estimate 650 for a specified atom (e.g., the alpha carbon atom) in each amino acid of the protein. The folding neural network 600 can generate the confidence estimate 650 for the specified atom in an amino acid in the protein, e.g., by processing the updated single embedding for the amino acid that is generated by the last update block in the folding neural network using one or more neural network layers, e.g., fully-connected layers.

The structure prediction system can generate a respective confidence score corresponding to each amino acid in the protein based on the confidence estimates 650 for the atoms in the predicted protein structure. For example, the structure prediction system can generate a confidence score for an amino acid as the expected value of a probability distribution over possible values of the accuracy score for the alpha carbon atom in the amino acid.

The structure prediction system can generate a confidence score for the entire predicted structure, e.g., as an average of the confidence scores for the amino acids in the protein.

During training of the structure prediction system, a training engine can adjust the parameter values of the structure prediction system by backpropagating gradients of an auxiliary loss that measures an error between: (i) confidence estimates generated by the folding neural network 600, and (ii) accuracy scores generated by comparing the predicted protein structure to the ground truth protein structure. The error may be, e.g., a cross-entropy error.

Confidence estimates generated by structure prediction systems can be used in a variety of ways. For example, confidence estimates for atoms in the predicted protein structure can indicate which parts of the structure have been reliably estimated are therefore suitable for further downstream processing or analysis. As another example, per-protein confidence scores can be used to rank a set of predictions for the structure of a protein, e.g., that have been generated by the same structure prediction system by processing different inputs characterizing the same protein, or that have been generated by different structure prediction systems.

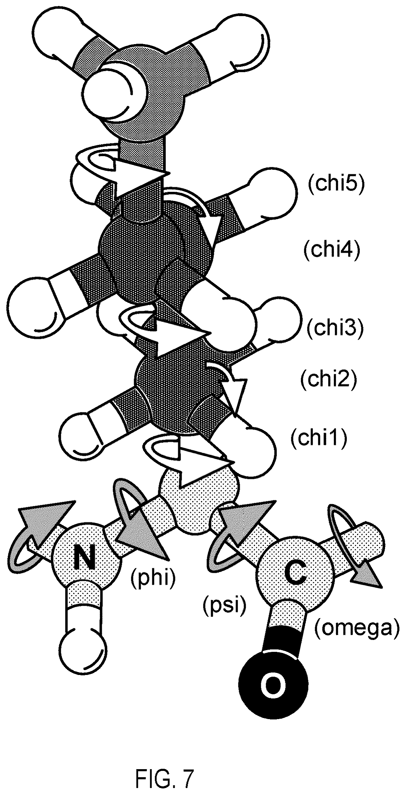

The location and rotation parameters specified by the structure parameters 106 can define the spatial locations (e.g., in *[x, y,* z] Cartesian coordinates) of the main chain atoms in the amino acids of the protein. However, the structure parameters 106 do not necessarily define the spatial locations of the remaining atoms in the amino acids of the protein, e.g., the atoms in the side chains of the amino acids. In particular, the spatial locations of the remaining atoms in an amino acid depend on the values of the torsion angles between the bonds in the amino acid, e.g., the omega-angle, the phi-angle, the psi-angle, the chi1-angle, the chi2-angle, the chi3-angle, and the chi4 angle, as illustrated with reference to FIG. 7.

Optionally, one or more of the update blocks 610 of the folding neural network 600 can generate an output that defines a respective predicted spatial location for each atom in each amino acid of the protein. To generate the predicted spatial locations for the atoms in an amino acid, the update block can process the updated single embedding for the amino acid using one or more neural network layers to generate predicted values of the torsion angles of the bonds between the atoms in the amino acid. The neural network layers may be, e.g., fully-connected neural network layers embedded with residual connections. Each torsion angle may be represented, e.g., as a 2-D vector.

The update block can determine the spatial locations of the atoms in an amino acid based on: (i) the values of the torsion angles for the amino acid, and (ii) the updated structure parameters (e.g., location and rotation parameters) for the amino acid. For example, the update block can process the torsion angles in accordance with a predefined function to generate the spatial locations of the atoms in the amino acid in a local reference frame of the amino acid. The update block can generate the spatial locations of the atoms in the amino acid in a global reference frame (i.e., that is common to all the amino acids in the protein) by rotating and translating the spatial locations of the atoms in accordance with the updated structure parameters for the amino acid. For example, the update block can determine the spatial location of an atom in the global reference frame by applying the rotation operation defined by the updated rotation parameters to the spatial location of the atom in the local reference frame to generate a rotated spatial location, and then apply the translation operation defined by the updated location parameters to the rotated spatial location.

In some implementations, alternatively to or in combination with outputting the final structure parameters, the folding neural network 600 outputs the predicted spatial locations of the atoms in the amino acids of the protein that are generated by the final update block.

The folding neural network 600 described with reference to FIG. 6 is characterized herein as receiving an input that is based on an MSA representation 114 and pair embeddings 116 that are generated by an embedding neural network, e.g., as described with reference to FIG. 2. In general, however, the inputs to the folding neural network (e.g., the single embeddings 602 and the pair embeddings 116) can be generated using any appropriate technique. Moreover, various aspects of the operations performed by the folding neural network (e.g., predicting spatial locations for the atoms in each amino acid of the protein) can be performed by other folding neural networks, e.g., with different architectures that receive different inputs.

FIG. 7 illustrates the torsion angles between the bonds in the amino acid, e.g., the omega-angle, the phi-angle, the psi-angle, the chi1-angle, the chi2-angle, the chi3-angle, the chi4 angle, and the chi5 angle.

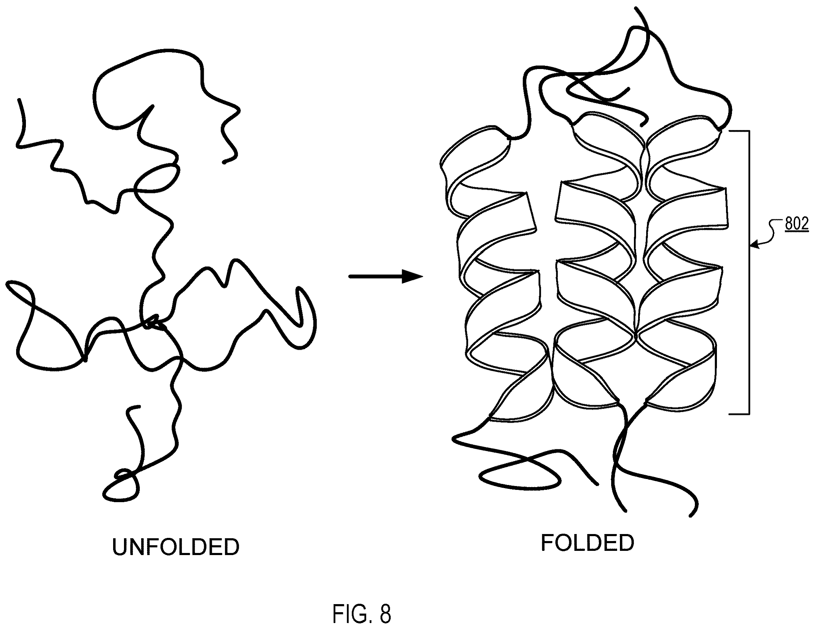

FIG. 8 is an illustration of an unfolded protein and a folded protein. The unfolded protein is a random coil of amino acids. The unfolded protein undergoes protein folding and folds into a 3D configuration. Protein structures often include stable local folding patterns such alpha helices (e.g., as depicted by 802) and beta sheets.

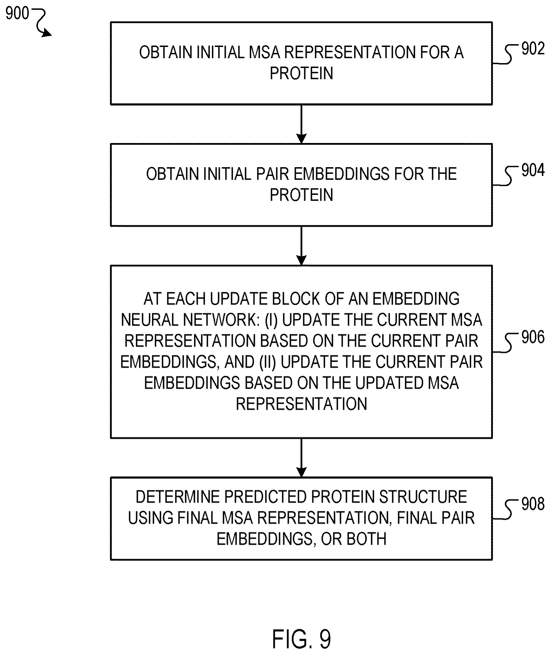

FIG. 9 is a flow diagram of an example process 900 for predicting the structure of a protein that includes one or more chains, where each chain specifies a sequence of amino acids. For convenience, the process 900 will be described as being performed by a system of one or more computers located in one or more locations. For example, a protein structure prediction system, e.g., the protein structure prediction system 100 of FIG. 1, appropriately programmed in accordance with this specification, can perform the process 900.

The system obtains an initial multiple sequence alignment (MSA) representation that represents a respective MSA corresponding to each chain in the protein (902).

The system obtains a respective initial pair embedding for each pair of amino acids in the protein (904).

The system processes an input including the initial MSA representation and the initial pair embeddings using an embedding neural network to generate an output that includes a final MSA representation and a respective final pair embedding for each pair of amino acids in the protein.

The embedding neural network includes a sequence of update blocks. Each update block has a respective set of update block parameters and is configured to receive a current MSA representation and a respective current pair embedding for each pair of amino acids in the protein. Each update block: (i) updates the current MSA representation based on the current pair embeddings, and (ii) updates the current pair embeddings based on the updated MSA representation (906).

The system determines a predicted structure of the protein based using the final MSA representation, the final pair embeddings, or both (908).

FIG. 10 shows an example process 1000 for generating a MSA representation 1010 for an amino acid chain in the protein. The protein structure prediction system 100, described with reference to FIG. 1, can implement the operations of the process 1000.

To generate the MSA representation 100 for the amino acid chain in the protein, the system 100 obtains a MSA 1002 for the protein that may include, e.g., thousands of MSA sequences.

The system 100 divides the set of MSA sequences into a set of "core" MSA sequences 1004 and a set of "extra" MSA sequences 1006. The set of core MSA sequences can be smaller (e.g., by an order of magnitude) than the set of extra MSA sequences 1006. The system 100 can divide the set of MSA sequences into core MSA sequences 1004 and extra MSA sequences 1006, e.g., by randomly selecting a predetermined number of the MSA sequences as core MSA sequences, and identifying the remaining MSA sequences as extra MSA sequences 1006.

For each extra MSA sequence 1006, the system 100 can determine a respective similarity measure (e.g., based on a Hamming distance) between the extra MSA sequence and each core MSA sequence 1004. The system 100 can then associate each extra MSA sequence 1006 with the corresponding core MSA sequence 1004 to which the extra MSA sequence 1006 is most similar (i.e., according to the similarity measure). The set of extra MSA sequences 1006 associated with a core MSA sequence 1004 can be referred to as a "MSA sequence cluster" 1008. That is, the system 100 determines a respective MSA sequence cluster 1008 corresponding to each core MSA sequence 1004, where the MSA sequence cluster 1008 corresponding to a core MSA sequence 1004 includes the set of extra MSA sequences 1006 that are most similar to the core MSA sequence 1004.

The system 100 can generate the MSA representation for the amino acid chain in the protein based on the core MSA sequences and the MSA sequence clusters 1008. The MSA representation 1010 can be represented by an $M \times N$ array of embeddings, where M is the number of core MSA sequences (i.e., such that each core MSA sequence is associated with a respective row of the MSA representation), and $N$ is the number of amino acids in the amino acid chain. The embeddings in the MSA representation can be indexed by $(i, j) \in \{(i, j): i = 1, ..., M, j = 1, ..., N\}$.

To generate the embedding at position $(i, j)$ in the MSA representation 1010, the system 100 can obtain an embedding (e.g., a one-hot embedding) defining the identity of the amino acid at position $j$ in core MSA sequence i. The system 100 can also determine a probability distribution over the set of possible amino acids based on the relative frequency of occurrence of each possible amino acid at position $j$ in the extra MSA sequences 1006 in the MSA sequence cluster 1008 corresponding to core MSA sequence i. The system 100 can then determine the embedding at position $(i, j)$ in the MSA representation by combining (e.g., concatenating): (i) the embedding defining the identity of the amino acid at position $j$ in core MSA sequence $i$, and (ii) the probability distribution over possible amino acids corresponding to position $j$ in core MSA sequence $i$.

In some cases, the (ground truth) protein structure may be known for one or more of the core MSA sequences. In particular, for one or more of the core MSA sequences, the values of the torsion angles between the bonds in the amino acids in the core MSA sequence (e.g., the omega-angle, the phi-angle, the psi-angle, etc.) may be known. If the values of the torsion angles for the amino acids in core MSA sequence i are known, then the system 100 can generate the embedding at position $(i,j)$ in the MSA representation based at least in part on the values of the torsion angles for amino acid $j$ in core MSA sequence $i$. For example, the system can generate an embedding of the values of the torsion angles using one or more neural network layers, and the concatenate the embedding of the values of the torsion angles to the embedding at position $(i, j)$ in the MSA representation.

FIG. 11 shows an example process 1100 for generating (initializing) a respective pair embedding 112 for each pair of amino acids in the protein. The protein structure prediction system 100, described with reference to FIG. 1, can implement the operations of the process 1100.

The system 100 can generate the pair embeddings 112 using an MSA representation 1102 of the protein. Generating a MSA representation for the protein is described in more detail with reference to FIG. 1 and FIG. 10. The system 100 can generate the MSA representation 1102 for the protein based on a respective MSA representation for each amino acid chain in the protein. To generate the MSA representation for an amino acid chain in the protein, the system 100 can use more MSA sequences (e.g., by an order of magnitude) than were used to generate the MSA representation 110 described with reference to FIG. 1. Therefore, the MSA representation 1102 used by the system 100 to generate the pair embeddings 112 may have more rows (e.g., by an order of magnitude) than the MSA representation 110 described with reference to FIG. 1. In some implementations, the system 100 can use the extra MSA sequences 1006 described with reference to FIG. 10 to generate the MSA representation 1102.

After generating the MSA representation 1102, the system 100 processes the MSA representation 1102 to generate pair embeddings 1104 from the MSA representation 1102, e.g., by applying an outer product mean operation to the MSA representation 1102, and identifying the pair embeddings 1104 as the result of the outer product mean operation.

The system 100 processes the MSA representation 1102 and the pair embeddings 1104 using an embedding neural network 1106. The embedding neural network 1106 can update the MSA representation 1102 and the pair embeddings 1104 by sharing information between the MSA representation 1102 and the pair embeddings 1104. More specifically, the embedding neural network 1106 can alternate between updating the MSA representation 1102 based on the pair embeddings 1104, and updating the pair embeddings 1104 based on the MSA representation 1102.

The embedding neural network 1106 can have an architecture based on the embedding neural network architecture described with reference to FIGS. 2-5, i.e., that updates the pair embeddings 1104 and the MSA representation 1102 using row-wise and column-wise self-attention blocks.

In some implementations, the embedding neural network 1106 can update the embeddings in each column of the MSA representation 1102 using a column-wise "global" self-attention operation. More specifically, the embedding neural network 1106 can provide the embeddings in each column of the MSA representation to a column-wise global self-attention block to generate updated embeddings for each column of the current MSA representation. To implement global column-wise self-attention, the self-attention block can generate a respective query embedding for each embedding in a column, and then average the query embeddings to generate a single "global" query embedding for the column. The column-wise self-attention block then uses the single global query embedding to perform the self-attention operation, which can reduce the complexity of the self-attention operation from quadratic (i.e., in the number of embeddings per column) to linear. Using a global self-attention operation can reduce the computational complexity of the column-wise self-attention operation to enable the column-wise self-attention operation to be performed on columns of the MSA representation 1102 that include large numbers (e.g., thousands) of embeddings.

After updating the pair embeddings 1104 and the MSA representation 1102 using the embedding neural network 1106, the system 100 can identify the pair embeddings 112 as the updated pair embeddings generated by the embedding neural network 1106. The system 100 can discard the updated MSA representation generated by the embedding neural network 1106, or use it any appropriate way.

As part of generating the pair embeddings 112, the system 100 can include relative position encoding information in the respective pair embedding corresponding to each pair of amino acids in the protein. The system can include the relative position encoding information for a pair of amino acids that are included in the same amino acid chain in the corresponding pair embedding by: computing the signed difference representing the number of amino acids separating the pair of amino acids in the amino acid chain, clipping the result to a predefined interval, representing the clipped value using a one-hot encoding vector, applying a linear transformation to the one-hot encoding vector, and adding the result of the linear transformation to the corresponding pair embedding. The system can include the relative position encoding information for a pair of amino acids that are not included in the same amino acid chain in the corresponding pair embedding by adding a default encoding vector to the corresponding pair embedding which indicates that the pair of amino acids are not included in the same amino acid chain.

The system 100 can also generate the pair embeddings 112 based at least in part on a set of one or more template sequences 1110. Each template sequence 1110 is an MSA sequence for an amino acid chain in the protein where the folded structure of the template sequence 1110 is known, e.g., from physical experiments.

The system 100 can generate a respective template representation 1112 of each template sequence 1110. A template representation 1112 of a template sequence 1110 includes a respective embedding corresponding to each pair of amino acids in the template sequence, e.g., such that a template representation 1112 of a template sequence of length $n$ (i.e., with $n$ amino acids) can be represented as an $n \times n$ array of embeddings. The system 100 generate the embedding at position $(i,j)$ in the template representation 1112 of a template sequence 1110 based on, e.g.: (i) respective embeddings (e.g., one-hot embeddings) representing the identities of the amino acid at position $i$ and position $j$ in the template sequence, (ii) a unit vector defined by the difference in spatial positions of the respective carbon alpha atoms in the amino acids at position $i$ and $j$ in the template sequence, i.e., in the folded structure of the template sequence, where the unit vector is computed in the frame of reference of amino acid $i$ or amino acid $j$, and (iii) a discretized/binned representation of the distance between the spatial positions of the respective carbon alpha atoms in the amino acids at position $i$ and $j$ in the template sequence.

The system 100 can process each template representation using a sequence of one or more template update blocks 1114 to generate a respective updated template representation 1116 corresponding to each template sequence 1110. The template update blocks can include, e.g., row-wise self-attention blocks (e.g., that update the embeddings in each row of the template representations), column-wise self-attention blocks (e.g., that update the embeddings in each column of the template representations), and transition blocks (e.g., that apply one or more neural network layers to each of the embeddings in the template representations).

After generating the updated template representations 1116, the system 100 uses the updated template representations 1116 to update the pair embeddings 112. For example, the system 100 can update the respective pair embedding 112 at each position $(i, j)$ using "cross-attention" over the embeddings at the corresponding $(i,j)$ positions of the updated template representations 1116. In a cross-attention operation to update the pair embedding 112 at position $(i,j)$, the query embedding is generated from the pair embedding at position $(i,j)$, and the key and value embeddings are generated from the embeddings at the corresponding $(i,j)$ positions of the updated template representations 1116.

Updating the pair embeddings 112 using the template sequences 1110 enables the system 100 to enrich the pair embeddings with information characterizing the protein structures of the evolutionarily related template sequences 1110, thereby enhancing the information content of the pair embeddings 112, and improving the accuracy of protein structures predicted using the pair embeddings.

This specification uses the term "configured" in connection with systems and computer program components. For a system of one or more computers to be configured to perform particular operations or actions means that the system has installed on it software, firmware, hardware, or a combination of them that in operation cause the system to perform the operations or actions. For one or more computer programs to be configured to perform particular operations or actions means that the one or more programs include instructions that, when executed by data processing apparatus, cause the apparatus to perform the operations or actions.

Embodiments of the subject matter and the functional operations described in this specification can be implemented in digital electronic circuitry, in tangibly-embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible non-transitory storage medium for execution by, or to control the operation of, data processing apparatus. The computer storage medium can be a machine-readable storage device, a machine-readable storage substrate, a random or serial access memory device, or a combination of one or more of them. Alternatively or in addition, the program instructions can be encoded on an artificially-generated propagated signal, e.g., a machine-generated electrical, optical, or electromagnetic signal, that is generated to encode information for transmission to suitable receiver apparatus for execution by a data processing apparatus.

The term "data processing apparatus" refers to data processing hardware and encompasses all kinds of apparatus, devices, and machines for processing data, including by way of example a programmable processor, a computer, or multiple processors or computers. The apparatus can also be, or further include, special purpose logic circuitry, e.g., an FPGA (field programmable gate array) or an ASIC (application-specific integrated circuit). The apparatus can optionally include, in addition to hardware, code that creates an execution environment for computer programs, e.g., code that constitutes processor firmware, a protocol stack, a database management system, an operating system, or a combination of one or more of them.

A computer program, which may also be referred to or described as a program, software, a software application, an app, a module, a software module, a script, or code, can be written in any form of programming language, including compiled or interpreted languages, or declarative or procedural languages; and it can be deployed in any form, including as a stand-alone program or as a module, component, subroutine, or other unit suitable for use in a computing environment. A program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, sub-programs, or portions of code. A computer program can be deployed to be executed on one computer or on multiple computers that are located at one site or distributed across multiple sites and interconnected by a data communication network.

In this specification the term "engine" is used broadly to refer to a software-based system, subsystem, or process that is programmed to perform one or more specific functions. Generally, an engine will be implemented as one or more software modules or components, installed on one or more computers in one or more locations. In some cases, one or more computers will be dedicated to a particular engine; in other cases, multiple engines can be installed and running on the same computer or computers.

The processes and logic flows described in this specification can be performed by one or more programmable computers executing one or more computer programs to perform functions by operating on input data and generating output. The processes and logic flows can also be performed by special purpose logic circuitry, e.g., an FPGA or an ASIC, or by a combination of special purpose logic circuitry and one or more programmed computers.

Computers suitable for the execution of a computer program can be based on general or special purpose microprocessors or both, or any other kind of central processing unit. Generally, a central processing unit will receive instructions and data from a read-only memory or a random access memory or both. The essential elements of a computer are a central processing unit for performing or executing instructions and one or more memory devices for storing instructions and data. The central processing unit and the memory can be supplemented by, or incorporated in, special purpose logic circuitry. Generally, a computer will also include, or be operatively coupled to receive data from or transfer data to, or both, one or more mass storage devices for storing data, e.g., magnetic, magneto-optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device, e.g., a universal serial bus (USB) flash drive, to name just a few.

Computer-readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's device in response to requests received from the web browser. Also, a computer can interact with a user by sending text messages or other forms of message to a personal device, e.g., a smartphone that is running a messaging application, and receiving responsive messages from the user in return.

Data processing apparatus for implementing machine learning models can also include, for example, special-purpose hardware accelerator units for processing common and compute-intensive parts of machine learning training or production, i.e., inference, workloads.

Machine learning models can be implemented and deployed using a machine learning framework, e.g., a TensorFlow framework, a Microsoft Cognitive Toolkit framework, an Apache Singa framework, or an Apache MXNet framework.

Embodiments of the subject matter described in this specification can be implemented in a computing system that includes a back-end component, e.g., as a data server, or that includes a middleware component, e.g., an application server, or that includes a front-end component, e.g., a client computer having a graphical user interface, a web browser, or an app through which a user can interact with an implementation of the subject matter described in this specification, or any combination of one or more such back-end, middleware, or front-end components. The components of the system can be interconnected by any form or medium of digital data communication, e.g., a communication network. Examples of communication networks include a local area network (LAN) and a wide area network (WAN), e.g., the Internet.

The computing system can include clients and servers. A client and server are generally remote from each other and typically interact through a communication network. The relationship of client and server arises by virtue of computer programs running on the respective computers and having a client-server relationship to each other. In some embodiments, a server transmits data, e.g., an HTML page, to a user device, e.g., for purposes of displaying data to and receiving user input from a user interacting with the device, which acts as a client. Data generated at the user device, e.g., a result of the user interaction, can be received at the server from the device.

While this specification contains many specific implementation details, these should not be construed as limitations on the scope of any invention or on the scope of what may be claimed, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially be claimed as such, one or more features from a claimed combination can in some cases be excised from the combination, and the claimed combination may be directed to a subcombination or variation of a subcombination.

Similarly, while operations are depicted in the drawings and recited in the claims in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the embodiments described above should not be understood as requiring such separation in all embodiments, and it should be understood that the described program components and systems can generally be integrated together in a single software product or packaged into multiple software products.

Particular embodiments of the subject matter have been described. Other embodiments are within the scope of the following claims. For example, the actions recited in the claims can be performed in a different order and still achieve desirable results. As one example, the processes depicted in the accompanying figures do not necessarily require the particular order shown, or sequential order, to achieve desirable results. In some cases, multitasking and parallel processing may be advantageous.

Innovative aspects of the present disclosure are also set out in the following numbered clauses, which are not claims.

Clause 1. A method performed by one or more data processing apparatus for predicting a structure of a protein comprising one or more chains, wherein each chain comprises a sequence of amino acids, the method comprising:

obtaining an initial multiple sequence alignment (MSA) representation that represents a respective MSA corresponding to each chain in the protein;
obtaining a respective initial pair embedding for each pair of amino acids in the protein;
processing an input comprising the initial MSA representation and the initial pair embeddings using an embedding neural network to generate an output that comprises a final MSA representation and a respective final pair embedding for each pair of amino acids in the protein,
wherein the embedding neural network comprises a sequence of update blocks,
wherein each update block has a respective set of update block parameters and performs operations comprising:

receiving a current MSA representation and a respective current pair embedding for each pair of amino acids in the protein;
updating the current MSA representation, in accordance with values of the update block parameters of the update block, based on the current pair embeddings; and
updating the current pair embeddings, in accordance with the values of the update block parameters of the update block, based on the updated MSA representation; and

determining a predicted structure of the protein using the final MSA representation, the final pair embeddings, or both.

Clause 2. The method of clause 1, wherein the current MSA representation comprises a plurality of embeddings, and wherein updating the current MSA representation based on the current pair embeddings comprises:
updating the current MSA representation using attention over the embeddings in the MSA representation, wherein the attention is conditioned on the current pair embeddings.

Clause 3. The method of clause 2, wherein updating the current MSA representation using attention over the embeddings in the current MSA representation comprises:

generating, based on the current MSA representation, a plurality of attention weights;
generating, based on the current pair embeddings, a respective attention bias corresponding to each of the attention weights;
generating a plurality of biased attention weights based on the attention weights and the attention biases; and
updating the embeddings in the current MSA representation using attention over the embeddings in the current MSA representation based on the biased attention weights.

Clause 4. The method of clause 3, wherein updating the embeddings in the current MSA representation using attention based on the biased attention weights comprises, for each embedding in the current MSA representation:
updating the embedding, based on the biased attention weights, using attention over only embeddings in the MSA representation that are located in a same row as the embedding in an arrangement of the embeddings in the current MSA representation into a two-dimensional array.

Clause 5. The method of any preceding clause, wherein updating the current pair embeddings based on the updated MSA representation comprises:

applying a transformation operation to the updated MSA representation; and
updating the current pair embeddings by adding a result of transformation operation to the current pair

24

embeddings.

Clause 6. The method of clause 5, wherein the transformation operation comprises an outer product mean operation.

Clause 7. The method of any one of clauses 5-6, wherein updating the current pair embeddings based on the updated MSA representation further comprises, after adding the result of the transformation operation to the current pair embeddings:
updating the current pair embeddings using attention over the current pair embeddings, wherein the attention is conditioned on the current pair embeddings.

Clause 8. The method of clause 7, wherein updating the current pair embeddings using attention over the current pair embeddings comprises:

generating, based on the current pair embeddings, a plurality of attention weights;
generating, based on the current pair embeddings, a respective attention bias corresponding to each attention weight;
generating a plurality of biased attention weights based on the attention weights and the attention biases; and
updating the current pair embeddings using attention over the current pair embeddings based on the biased attention weights.

Clause 9. The method of clause 8, wherein updating the current pair embeddings using attention over the current pair embeddings based on the biased attention weights comprises, for each current pair embedding:
updating the current pair embedding, based on the biased attention weights, using attention over only current pair embeddings that are located in a same row as the current pair embedding in an arrangement of the current pair embeddings into a two-dimensional array.

Clause 10. The method of any one of clauses 8-9, wherein updating the current pair embeddings using attention over the current pair embeddings based on the biased attention weights comprises, for each current pair embedding:
updating the current pair embedding, based on the biased attention weights, using attention over only current pair embeddings that are located in a same column as the current pair embedding in an arrangement of the current pair embeddings into a two-dimensional array.

Clause 11. The method of any preceding clause, wherein the protein comprises a plurality of chains, and wherein obtaining the initial MSA representation that represents a respective multiple MSA corresponding to each chain in the protein comprises:

obtaining a respective representation of the MSA corresponding to each chain in the protein as a two-dimensional array of embeddings; and
assembling the representations of the MSAs corresponding to the chains into a block diagonal array.

Clause 12. The method of any preceding clause, wherein determining the predicted structure of the protein comprises processing an input comprising the final pair embeddings using a folding neural network to generate an output that defines the predicted structure of the protein, comprising, for each of a plurality of pairs of amino acids in the protein:
processing a final pair embedding for the pair of amino acids, in accordance with values of folding neural network parameters, to generate an output specifying a probability distribution over a set of possible distances between the pair of amino acids in a structure of the protein.

Clause 13. The method of any preceding clause, wherein determining the predicted structure of the protein comprises processing an input comprising the final pair embeddings using a folding neural network to generate an output that defines the predicted structure of the protein, comprising:

obtaining an initial single embedding and initial values of structure parameters for each amino acid in the protein, wherein the structure parameters for each amino acid comprise location parameters that specify a predicted three-dimensional spatial location of the amino acid in the structure of the protein;
processing a folding network input comprising the final pair embeddings, the initial embedding for each amino acid in the protein, and the initial values of the structure parameters for each amino acid in the protein using the folding neural network to generate a network output comprising final values of the structure parameters for each amino acid in the protein, wherein the folding neural network comprises a plurality of update blocks, wherein each update block comprises a plurality of neural network layers and is configured to:

receive an update block input comprising the final pair embeddings, a current single embedding for each amino acid in the protein, and current values of the structure parameters for each amino acid in the protein; and

process the update block input to update the current single embedding and the current values of the structure parameters for each amino acid in the protein;

wherein the final values of the structure parameters for each amino acid in the amino acid sequence collectively characterize the predicted structure of the protein.

Clause 14. The method of clause 13, wherein obtaining the initial single embedding for each amino acid in the protein comprises:
determining the initial single embedding for each amino acid in the protein based on the final MSA representation.

Clause 15. The method of any one of clauses 13-14, wherein the structure parameters for each amino acid further comprise rotation parameters that specify a predicted spatial orientation of the amino acid in the structure of the protein.

Clause 16. The method of clause 15, wherein the rotation parameters define a $3 \times 3$ rotation matrix.

Clause 17. The method of any one of clauses 15-16, wherein processing the update block input to update the current single embedding and the current values of the structure parameters for each amino acid in the protein comprises:

updating the current single embedding for each amino acid in the protein; and
updating the current values of the structure parameters for each amino acid in the protein based on the updated single embeddings for the amino acids in the protein.

Clause 18. The method of clause 17, wherein a final update block of the plurality of update blocks is further configured to generate an output that defines a predicted three-dimensional spatial location of each atom in each amino acid in the protein.

Clause 19. The method of clause 18, wherein generating an output that defines a respective three-dimensional spatial location of each atom in each amino acid in the protein comprises, for each amino acid:

processing the updated single embedding for the amino acid to generate a respective value of each of a plurality of torsion angles of bonds between the atoms in the amino acid; and
determining a spatial location of each atom in the amino acid in a local reference frame of the amino acid based on the values of the plurality of torsion angles.

Clause 20. The method of clause 19, further comprising, for each amino acid, determining a spatial location of each atom in the amino acid in a global reference frame of the protein based on: (i) the spatial locations of the atoms in the local reference frame of the amino acid, and (ii) the updated values of the structure parameters for the amino acid.

Clause 21. The method of any one of clauses 17-20, wherein updating the current single embedding for a target amino acid in the protein comprises:

determining a respective attention weight between the target amino acid and each source amino acid in the protein, comprising:

generating a three-dimensional query embedding of the target amino acid based on the current single embedding of the target amino acid;
generating, for each source amino acid in the protein, a three-dimensional key embedding of the source amino acid based on the current single embedding of the source amino acid; and
determining the attention weight between the target amino acid and each source amino acid in the protein based at least in part on a difference between: (i) the three-dimensional query embedding of the target amino acid, and (ii) the three-dimensional key embedding of the source amino acid; and

updating the current single embedding of the target amino acid using the attention weights.

Clause 22. The method of clause 21, wherein generating the three-dimensional query embedding of the target amino acid based on the current single embedding of the target amino acid comprises:

processing the current single embedding of the target amino acid using a linear neural network layer that generates a three-dimensional output; and
applying a rotation operation and a translation operation to the three-dimensional output of the linear neural network layer, wherein the rotation operation is specified by the current values of the rotation parameters for the target amino acid and the translation operation is specified by the current values of the location parameters for the target amino acid.

Clause 23. The method of any one of clauses 21-22, wherein generating a three-dimensional key embedding of the source amino acid based on the current single embedding of the source amino acid comprises:

processing the current single embedding of the source amino acid using a linear neural network layer that generates a three-dimensional output; and
applying a rotation operation and a translation operation to the three-dimensional output of the linear neural network layer, wherein the rotation operation is specified by the current values of the rotation parameters for the source amino acid and the translation operation is specified by the current values of the location parameters for the source amino acid.

Clause 24. The method of any one of clauses 21-23, wherein determining the attention weight between the target amino acid and each source amino acid in the protein further comprises, for each source amino acid in the protein:

determining a projection of a final pair embedding corresponding to a pair of amino acids that comprises the target amino acid and the source amino acid; and
determining the attention weight between the target amino acid and the source amino acid based at least in part on the projection of the final pair embedding corresponding to the pair of amino acids that comprises the target amino acid and the source amino acid.

Clause 25. The method of any one of clauses 21-24, wherein updating the current single embedding of the target amino acid using the attention weights comprises:

generating, for each amino acid in the protein, a three-dimensional value embedding of the amino acid based on the current single embedding of the amino acid;
determining a weighted linear combination of the three-dimensional value embeddings of the amino acids using the attention weights;
generating a geometric return embedding by applying a rotation operation and a translation operation to the weighted linear combination, wherein the rotation operation inverts a rotation operation specified by the current values of the rotation parameters for the target amino acid and the translation operation is specified by a negative of the current values of the location parameters for the target amino acid; and
updating the current embedding of the target amino acid based on the geometric return embedding.

Clause 26. The method of any one of clauses 17-25, wherein updating the current values of the structure parameters for each amino acid in the protein based on the updated single embeddings for the amino acids in the protein comprises, for each amino acid:

determining updated values of the location parameters for the amino acid as a sum of: (i) the current values of the location parameters for the amino acid, and (ii) a linear projection of the updated single embedding of the amino acid;
determining updated values of the rotation parameters for the amino acid as a composition of: (i) a rotation operation specified by the current values of the rotation parameters for the amino acid, and (ii) a rotation operation specified by a quaternion with real part 1 and imaginary part specified by a linear projection of the updated single embedding of the amino acid.

Clause 27. The method of any one of clauses 13-26, further comprising, for each amino acid in the protein:

processing the updated current single embedding for the amino acid that is generated by a final update block to generate a confidence estimate for a position of an atom in the amino acid in the predicted structure of the protein, wherein the confidence estimate defines a probability distribution over a range of possible accuracies for the position of the atom.

Clause 28. A system comprising:

one or more computers; and
one or more storage devices communicatively coupled to the one or more computers, wherein the one or more storage devices store instructions that, when executed by the one or more computers, cause the one or more computers to perform operations of the respective method of any one of clauses 1-27.

Clause 29. One or more non-transitory computer storage media storing instructions that when executed by one or more computers cause the one or more computers to perform operations of the respective method of any one of clauses 1-27.

Clause 30. A method of obtaining a ligand, wherein the ligand is a drug or a ligand of an industrial enzyme, the method comprising:

performing the method of any one of clauses 1-27 to determine a predicted structure of a target protein;
evaluating an interaction of one or more candidate ligands with the predicted structure of the target protein;
and selecting one or more of the candidate ligands as the ligand dependent on a result of the evaluating.

Clause 31. The method of clause 30 wherein the target protein comprises a receptor or enzyme, and wherein the ligand is an agonist or antagonist of the receptor or enzyme.

Clause 32. The method of clause 30 or 31 wherein the ligand is a drug, and the method comprises:

performing the method of any one of clauses 1-27 to determine a predicted structure of each of a plurality of target proteins;
evaluating the interaction of the one or more candidate ligands with the predicted structure of each of the target proteins; and
selecting one or more of the candidate ligands as the ligand to either i) obtain a ligand that interacts with each of the target proteins, or ii) obtain a ligand that interacts with only one of the target proteins.

Clause 33. A method of obtaining a polypeptide ligand, wherein the ligand is a drug or a ligand of an industrial enzyme, the method comprising

for each of one or more candidate polypeptide ligands performing the method of any one of clauses 1-27 to determine a predicted structure of the candidate polypeptide ligand;
obtaining a target protein structure of a target protein;
evaluating an interaction between the predicted structure of each of the one or more candidate polypeptide ligands and the target protein structure; and
selecting one of the one or more of the candidate polypeptide ligands as the polypeptide ligand dependent on a result of the evaluating.

Clause 34. The method of clause 33 wherein the target protein comprises a receptor or enzyme, and wherein the ligand is an agonist or antagonist of the receptor or enzyme; or wherein the polypeptide ligand comprises an antibody and the target protein comprises an antibody target, in particular a virus or cancer cell protein, and wherein the antibody binds to the antibody target to provide a therapeutic effect.

Clause 35. A method of obtaining a diagnostic antibody marker of a disease, the method comprising:

for each of one or more candidate antibodies performing the method of any one of clauses 1-27 to determine a predicted structure of the candidate antibody;
obtaining a target protein structure of a target protein;
evaluating an interaction between the predicted structure of each of the one or more candidate antibodies and the target protein structure; and

selecting one of the one or more of the candidate antibodies as the diagnostic antibody marker dependent on a result of the evaluating.

Clause 36. The method of any one of clauses 30-35 wherein the evaluating the interaction of one of the candidate ligands comprises determining an interaction score for the candidate ligand, wherein the interaction score comprises a measure of an interaction between the candidate ligand and the target protein.

Clause 37. The method of any one of clauses 30-36 further comprising synthesizing the ligand.

Clause 38. The method of clause 37 further comprising testing biological activity of the ligand in vitro and in vivo.

Clause 39. A method of identifying the presence of a protein mis-folding disease, comprising:

performing the method of any one of clauses 1-27 to determine a predicted structure of a protein;
obtaining a structure of a version of the protein obtained from a human or animal body;

comparing the predicted structure of the protein with the structure of a version of the protein obtained from a human or animal body; and
identifying the presence of a protein mis-folding disease dependent upon a result of the comparison.

Clause 40. A system for predicting the structure of a protein comprising one or more chains, wherein each chain comprises a sequence of amino acids, the system comprising:

one or more computers; and
one or more storage devices communicatively coupled to the one or more computers, wherein the one or more storage devices store instructions that, when executed by the one or more computers, cause the one or more computers to implement:

an embedding neural network that is configured to perform operations comprising:

receiving an initial multiple sequence alignment (MSA) representation that represents a respective MSA corresponding to each chain in the protein;
receiving a respective initial pair embedding for each pair of amino acids in the protein;
processing the initial MSA representation and the initial pair embeddings to generate an output that comprises a final MSA representation and a respective final pair embedding for each pair of amino acids in the protein,
wherein the embedding neural network comprises a sequence of update blocks,
wherein each update block has a respective set of update block parameters and is configured to perform operations comprising:

receiving a current MSA representation and a respective current pair embedding for each pair of amino acids in the protein;
updating the current MSA representation, in accordance with values of the update block parameters of the update block, based on the current pair embeddings; and
updating the current pair embeddings, in accordance with the values of the update block parameters of the update block, based on the updated MSA representation; and

a folding neural network that is configured to perform operations comprising:

receiving a folding network input comprising the final MSA representation, the final pair embeddings, or both; and
processing the folding network input to generate a predicted structure of the protein.

**Claims**

1. A method performed by one or more computers for determining a predicted structure of a protein and for obtaining a ligand, wherein the ligand is a drug or a ligand of an industrial enzyme, the method comprising:

obtaining an initial single embedding and initial values of structure parameters for each amino acid in the protein, wherein the structure parameters for each amino acid comprise location parameters that specify a predicted three-dimensional spatial location of the amino acid in the structure of the protein;

processing a folding network input comprising the initial embedding for each amino acid in the protein and the initial values of the structure parameters for each amino acid in the protein using a folding neural network to generate a network output comprising final values of the structure parameters for each amino acid in the protein, wherein the folding neural network comprises a plurality of update blocks, wherein each update block comprises a plurality of neural network layers and is configured to:

receive an update block input comprising the final pair embeddings, a current single embedding for each amino acid in the protein, and current values of the structure parameters for each amino acid in the protein; and

process the update block input to update the current single embedding and the current values of the structure parameters for each amino acid in the protein;

wherein the final values of the structure parameters for each amino acid in the protein collectively characterize the predicted structure of the protein; and

evaluating an interaction of one or more candidate ligands with the predicted structure of the protein and selecting one or more of the candidate ligands as the ligand dependent on a result of the evaluating.

2. The method of claim 1, wherein the structure parameters for each amino acid further comprise rotation parameters that specify a predicted spatial orientation of the amino acid in the structure of the protein.

3. The method of claim 2, wherein the rotation parameters define a $3 \times 3$ rotation matrix.

4. The method of any one of claims 2-3, wherein processing the update block input to update the current single embedding and the current values of the structure parameters for each amino acid in the protein comprises:

updating the current single embedding for each amino acid in the protein; and

updating the current values of the structure parameters for each amino acid in the protein based on the updated single embeddings for the amino acids in the protein.

5. The method of claim 4, wherein a final update block of the plurality of update blocks is further configured to generate an output that defines a predicted three-dimensional spatial location of each atom in each amino acid in the protein.

6. The method of claim 5, wherein generating an output that defines a respective three-dimensional spatial location of each atom in each amino acid in the protein comprises, for each amino acid:

processing the updated single embedding for the amino acid to generate a respective value of each of a plurality of torsion angles of bonds between the atoms in the amino acid; and

determining a spatial location of each atom in the amino acid in a local reference frame of the amino acid based on the values of the plurality of torsion angles.

7. The method of claim 6, further comprising, for each amino acid, determining a spatial location of each atom in the amino acid in a global reference frame of the protein based on: (i) the spatial locations of the atoms in the local reference frame of the amino acid, and (ii) the updated values of the structure parameters for the amino acid.

8. The method of any one of claims 4-7, wherein updating the current single embedding for a target amino acid in the protein comprises:

determining a respective attention weight between the target amino acid and each source amino acid in the protein, comprising:

generating a three-dimensional query embedding of the target amino acid based on the current single embedding of the target amino acid;

generating, for each source amino acid in the protein, a three-dimensional key embedding of the source amino acid based on the current single embedding of the source amino acid; and

determining the attention weight between the target amino acid and each source amino acid in the protein based at least in part on a difference between: (i) the three-dimensional query embedding of the target amino acid, and (ii) the three-dimensional key embedding of the source amino acid; and

updating the current single embedding of the target amino acid using the attention weights.

9. The method of claim 8, wherein generating the three-dimensional query embedding of the target amino acid based on the current single embedding of the target amino acid comprises:

processing the current single embedding of the target amino acid using a linear neural network layer that generates a three-dimensional output; and
applying a rotation operation and a translation operation to the three-dimensional output of the linear neural network layer, wherein the rotation operation is specified by the current values of the rotation parameters for the target amino acid and the translation operation is specified by the current values of the location parameters for the target amino acid.

10. The method of any one of claims 8-9, wherein generating a three-dimensional key embedding of the source amino acid based on the current single embedding of the source amino acid comprises:

processing the current single embedding of the source amino acid using a linear neural network layer that generates a three-dimensional output; and
applying a rotation operation and a translation operation to the three-dimensional output of the linear neural network layer, wherein the rotation operation is specified by the current values of the rotation parameters for the source amino acid and the translation operation is specified by the current values of the location parameters for the source amino acid.

11. The method of any one of claims 8-10, wherein determining the attention weight between the target amino acid and each source amino acid in the protein further comprises, for each source amino acid in the protein:

determining a projection of a final pair embedding corresponding to a pair of amino acids that comprises the target amino acid and the source amino acid; and
determining the attention weight between the target amino acid and the source amino acid based at least in part on the projection of the final pair embedding corresponding to the pair of amino acids that comprises the target amino acid and the source amino acid.

12. The method of any one of claims 8-11, wherein updating the current single embedding of the target amino acid using the attention weights comprises:

generating, for each amino acid in the protein, a three-dimensional value embedding of the amino acid based on the current single embedding of the amino acid;
determining a weighted linear combination of the three-dimensional value embeddings of the amino acids using the attention weights;
generating a geometric return embedding by applying a rotation operation and a translation operation to the weighted linear combination, wherein the rotation operation inverts a rotation operation specified by the current values of the rotation parameters for the target amino acid and the translation operation is specified by a negative of the current values of the location parameters for the target amino acid; and
updating the current embedding of the target amino acid based on the geometric return embedding.

13. The method of any preceding claim, wherein the folding network input further comprises a respective final pair embedding for each pair of amino acids in the protein;
wherein the final pair embeddings have been generated by performing operations comprising:

obtaining an initial protein representation;
obtaining a respective initial pair embedding for each pair of amino acids in the protein;
processing an input comprising the initial protein representation and the initial pair embeddings using an embedding neural network to generate an output that comprises a final protein representation and a respective final pair embedding for each pair of amino acids in the protein,
wherein the embedding neural network comprises a sequence of update blocks,
wherein each update block has a respective set of update block parameters and performs operations comprising:

receiving a current protein representation and a respective current pair embedding for each pair of amino acids in the protein;

updating the current protein representation, in accordance with values of the update block parameters of the update block, based on the current pair embeddings; and

updating the current pair embeddings, in accordance with the values of the update block parameters of the update block, based on the updated protein representation.

14. A system comprising:

one or more computers; and

one or more storage devices communicatively coupled to the one or more computers, wherein the one or more storage devices store instructions that, when executed by the one or more computers, cause the one or more computers to perform operations of the respective method of any one of claims 1-13.

15. One or more non-transitory computer storage media storing instructions that when executed by one or more computers cause the one or more computers to perform operations of the respective method of any one of claims 1-13.

# PROTEIN STRUCTURE PREDICTION SYSTEM
## 100

PROTEIN 104

AMINO ACID CHAINS 102

MSA REPRESENTATION 110

PAIR EMBEDDINGS 112

EMBEDDING NEURAL NETWORK 200

UPDATED MSA REPRESENTATION 114

UPDATED PAIR EMBEDDINGS 116

FOLDING NEURAL NETWORK 600

STRUCTURE PARAMETERS 106

PREDICTED PROTEIN STRUCTURE 108

FIG. 1

# EMBEDDING NEURAL NETWORK <u>200</u>

FIG. 2

# EMBEDDING NEURAL NETWORK UPDATE BLOCK

FIG. 3

# MSA UPDATE BLOCK 400

FIG. 4

**PAIR UPDATE BLOCK 500**

CURRENT PAIR EMBEDDINGS 304

UPDATED MSA REPRESENTATION 306

OUTER PRODUCT MEAN 502

CONDITIONED ON PAIR EMBEDDINGS

ROW-WISE SELF-ATTENTION BLOCK 504

CONDITIONED ON PAIR EMBEDDINGS

COLUMN-WISE SELF-ATTENTION BLOCK 506

TRANSITION BLOCK 508

UPDATED PAIR EMBEDDING 308

FIG. 5

# FOLDING NEURAL NETWORK 600

```
                    ┌─────────────────┐
                    │      MSA        │
                    │ REPRESENTATION  │
                    │      114        │
                    └─────────────────┘
                             │
                             ▼
        INITIAL SINGLE              INITIAL STRUCTURE
        EMBEDDINGS 602              PARAMETERS 604
```

CURRENT SINGLE EMBEDDINGS 606

CURRENT STRUCTURE PARAMETERS 608

PAIR EMBEDDINGS 116

GEOMETRIC ATTENTION BLOCK 616

FOLDING BLOCK 618

UPDATE BLOCK 610

UPDATED SINGLE EMBEDDINGS 612

UPDATED STRUCTURE PARAMETERS 614

FINAL STRUCTURE PARAMETERS 106

CONFIDENCE ESTIMATES 650

PREDICTED PROTEIN STRUCTURE 108

FIG. 6

(chi5)

(chi4)

(chi3)

(chi2)

(chi1)

(phi)

(psi)

(omega)

N

C

O

FIG. 7

FIG. 8

900

OBTAIN INITIAL MSA REPRESENTATION FOR A PROTEIN — 902

OBTAIN INITIAL PAIR EMBEDDINGS FOR THE PROTEIN — 904

AT EACH UPDATE BLOCK OF AN EMBEDDING NEURAL NETWORK: (I) UPDATE THE CURRENT MSA REPRESENTATION BASED ON THE CURRENT PAIR EMBEDDINGS, AND (II) UPDATE THE CURRENT PAIR EMBEDDINGS BASED ON THE UPDATED MSA REPRESENTATION — 906

DETERMINE PREDICTED PROTEIN STRUCTURE USING FINAL MSA REPRESENTATION, FINAL PAIR EMBEDDINGS, OR BOTH — 908

FIG. 9

# GENERATING A MSA REPRESENTATION FOR AN AMINO ACID CHAIN IN THE PROTEIN

1000

```
              ┌──────────────────┐
              │    MSA 1002      │
              └──────────────────┘
                        │
          ┌─────────────┴─────────────┐
          ▼                           ▼
┌──────────────────┐        ┌──────────────────┐
│    CORE MSA      │        │    EXTRA MSA     │
│ SEQUENCES 1004   │        │ SEQUENCES 1006   │
└──────────────────┘        └──────────────────┘
          │                           │
          │                           ▼
          │                 ┌──────────────────┐
          │                 │  MSA SEQUENCE    │
          │                 │  CLUSTERS 1008   │
          │                 └──────────────────┘
          │                           │
          ▼                           ▼
        ┌──────────────────────────────┐
        │             MSA              │
        │        REPRESENTATION        │
        │             1010             │
        └──────────────────────────────┘
```

FIG. 10

# GENERATING INITIAL PAIR EMBEDDINGS

1100

MSA REPRESENTATION 1102

PAIR EMBEDDINGS 1104

EMBEDDING NEURAL NETWORK 1106

PAIR EMBEDDINGS 112

TEMPLATE SEQUENCES 1110

TEMPLATE REPRESENTATIONS 1112

TEMPLATE UPDATE BLOCKS 1114

UPDATED TEMPLATE REPRESENTATIONS 1116

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63118917
- US 63187362

**Non-patent literature cited in the description**

- **JIMMY LEI BA et al.** Layer Normalization. *arXiv:1607.06450*
- **J.L. BA** ; **J.R. KIROS** ; **G.E. HINTON**. Layer Normalization. *arXiv:1607.06450*, 2016
- **V. MARIANI et al.** lDDT: a local superposition-free score for comparing protein structures and models using distance difference tests. *Bioinformatics*, 01 November 2013, vol. 29 (21), 2722-2728